# EUROPEAN PATENT SPECIFICATION

(11) **EP 2 128 160 B1**
(45) Date of publication and mention of the grant of the patent: **14.07.2010**
(21) Application number: 08008254.8
(22) Date of filing: 30.04.2008
(51) Int. Cl.: C07D 471/04, A61K 31/437, A61K 31/4375, A61P 31/00, A61P 31/12, A61P 35/00

(54) **Indolo[2,3-b]-, Indeno[1,2-b]- and Indeno[2,1-b]pyrido[2,3-f] quinoxaline-3-carboxylic acids and esters, processes for their preparation and their use as antiviral, antibiotic and antitumor agents**
Indol[2,3-b]-, Inden[1,2-b]- und Inden[2,1-b]pyrido[2,3-f] Quinoxalin-3-Carboxylsäuren und Ester, Verfahren zu ihrer Herstellung und ihre Verwendung als Antivirus- und Antitumormittel
Acides et esters Indolo[2,3-b]-, Indeno[1,2-b]- and Indeno[2,1-b]pyrido[2,3-f] quinoxaline-3-carboxyliques, leurs processus de préparation et leur utilisation en tant qu'agents antiviraux, antibiotiques et antitumeurs

(43) Date of publication of application: 02.12.2009
(73) Proprietor: Universität Duisburg-Essen, 45141 Essen (DE)
(72) Inventor: Boese, Roland, 45326 Essen (DE); El-Abadelah, Mustafa M., Amman 11942 (JO); Zahra, Jalal A., Dr., Amman 11942 (JO)
(74) Representative: Bendele, Tanja

(56) References cited:
- EP-A- 0 350 733
- WO-A-87/04436
- "Römpp Chemie Lexikon" 1995, GEORG THEME VERLAG , STUTTGART, GERMANY * page 3557 *

## Description

The present invention relates to novel quinoxaline derivatives represented by regioisomeric structures of quinoxalines of general formulas I and/or II, 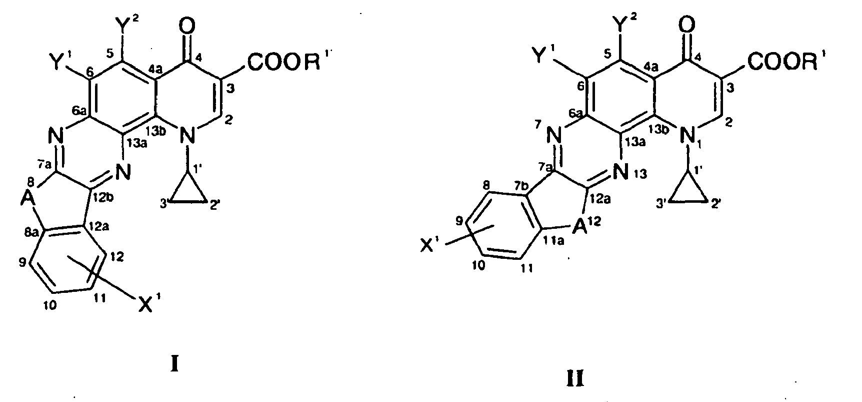 wherein the group COOR¹ independently is an acid, an ester and/or a salt, in particular R¹ is H, linear, branched or cyclic C₁-C₆-alkyl, in particular methyl, ethyl, iso-propyl, n-propyl, butyl, n-butyl, iso-butyl and/or tert-butyl, Y¹ and/or Y² are independently halogen and/or H, in particular F, Cl, Br or J; A is oxo (C=O), C(R³)₂ and/or N-R², wherein R³ and/or R² independently are H, a linear, branched or cyclic unsubstituted or substituted hydrocarbon, in particular a linear, branched or cyclic C₁-C₆-alkyl or C₁-C₆-alkylamin and X¹ is H, halogen, in particular F, Cl, Br or I; a process for the production of the quinoxalines of formulas I and II, a key precursor for the synthesis of quinoxalines of general formulas I and II as well as their use as pharmaceutical active agents, in particular as agent that is active against cancer and/or at least one virus or as an antibiotic.

Other quinoxaline derivatives are known to exhibit potent antiviral activity [WO 87/04436, WO 2007/084073] against e.g. *Herpes Simplex* virus type 1 (*HSV-1*), *Cytomegalo* virus (*CMV*) and *Vericella Zoster* virus (*VZV*). WO 87/04436 A1 discloses substituted indoloquinoxalines, e.g. 2,3-dimethyl-6-(N,N-dimethylaminoethyl)-6H-indolo(2,3-b)-quinoxaline, which have antiviral activity and have an effect against cancer. Compound **2** (referred to as **B-220**) and its congeners are believed to act via inhibition of the decapsidation process of the virus [Zegar I, Gräslund A, Bergman J, Eriksson M, Nordén B (1989) Chem Biol Interact 72: 277; Patel N, Bergman J, Gräslund A (1991) Eur J Biochem 197: 597].

The parent 6*H*-indolo[2,3-*b*]quinoxaline (**1**) was first synthesized in 1895 *via* cyclocondensation of isatin with o-phenylenediamine. An alternative synthetic route towards 1 (and derivatives thereof) involves initial interaction between *o-*phenylenediamines and 1-acetyl-2-bromo-3-indolinone. Derivatives of this tetracyclic heteroaromatic system are important *DNA* interchelators [Harmenberg J, Åkesson- Johansson A, Gräslund A, Malmfors T, Bergman J, Wahren B, Åkerfeldt S, Lundblad L, Cox S (1991) Antiviral Res 15: 205], some of which display antitumor activity [Arimondo PB, Baldeyrou B, Laine W, Bal C, Alphonse F-A, Routier S, Coudert G, Merour J-Y, Colson P, Houssier C, Bailly C (2001) Chem Biol Interact 138: 59], while others are useful agents for the treatment of autoimmune disease [US 2005/288,296; WO 2001/060371; WO 87/ 04436, WO 2007/084073] against e.g. *Herpes Simplex* virus type 1 (*HSV-1*), *Cytomegalo* virus (*CMV*) and *Vericella-Zoster* virus (*VZV*). 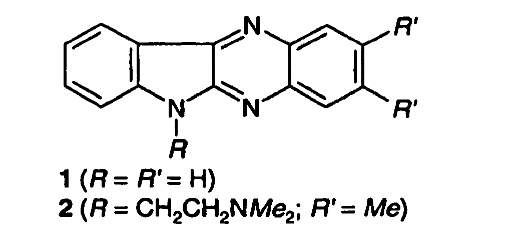 6*H*-Indolo[2,3-*b*]quinoxaline **1**, and its antiviral derivative **2**

Further antibacterial compounds are disclosed in EP 0 350733 A1 and EP 0757990 A1 which teach the use of 7-(1-pyrolidinyl)-3-chinolone- and - naphthyridonecarboxylic acid derivatives and their antibacterial use. EP 0 350 733 A2 discloses quinolone and naphthyridone derivatetives such as 7-(1-pyrrolidinyl)-3-quinolone- and -naphthyridone-carboxylic-acid derivatives, method for their preparation and substituted mono- and bi-cyclic pyrrolidine intermediates, and their antibacterial and feed additive compositions. These compounds are found to possess a good antibacterial action in the Gram-positive region.

The novel Quinoxaline derivative of the present invention represented by general formulas I or II possess pharmaceutical activity, in particular they are active as antibiotic and/or they are an anti cancer agent and/or an antiviral agent. 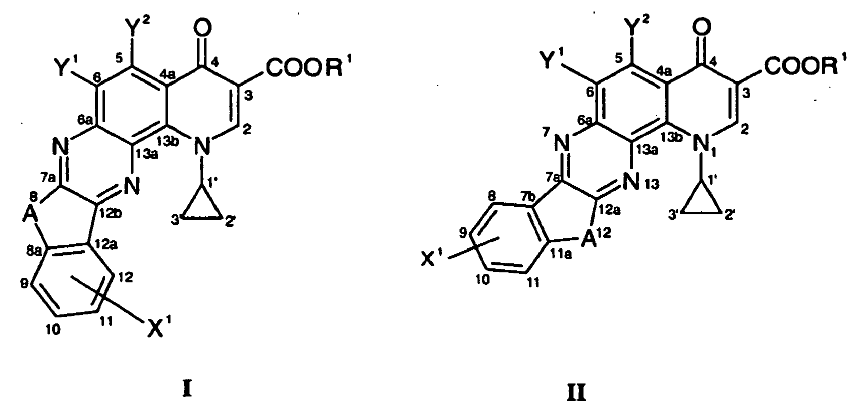 In general formulas I or II of the quinoxaline derivative of the present invention the group COOR¹ independently is an acid, an ester and/or a salt, in particular R¹ independently is H, a linear, branched or cyclic unsubstituted or substituted hydrocarbon, for example a linear, branched or cyclic C₁-C₆-alkyl, in particular methyl, ethyl, iso-propyl, n-propyl, butyl, n-butyl, iso-butyl, tert-butyl or cyclohexyl, as a salt R¹ is metal, e.g. a metal ion such as alkali and/or alkaline earth metal, as Na⁺, K⁺, Li⁺, Ca²⁺, Mg²⁺, or Zn²⁺;
- Y¹ and/or Y² are independently halogen and/or H, in particular F, Cl, Br or I, in particular F, Cl or H, especially F, most preferred Y¹ is F and Y² is H, Y¹ is H and Y² is F or Y¹ and Y² are both fluorine;
- A is oxo, a C=O group, a C(R³)₂ and/or a N-R² group, wherein each R³ and/or R² independently are H, a linear, branched or cyclic unsubstituted or substituted hydrocarbon, in particular R³ and/or R² independently are H, linear, branched or cyclic C₁-C₆-alkyl or C₁-C₁₂-alkylamin, such as -CH₂CH₂N(alkyl)₂ or -CH₂CH₂N(CH₃)₂ and
- X¹ is H, a linear, branched or cyclic unsubstituted or substituted hydrocarbon in particular X¹ is H, linear, branched or cyclic C₁-C₆-alkyl or C₁-C₁₂-alkylamin; or halogen, in particular F, Cl, Br or I and/or
- a polymorph or a solvate of the quinoxaline.

It is preferred that X¹ is located at C-9, C-10 or C-11, in particular at C-11. Formulas I and II are regioisomeric structures of the quinoxalines of the present invention.
Favoured are quinoxalines of formulas I and/or II in which R¹ is H, methyl, ethyl, a metal cation or an alkali metal cation, Y¹ is F, Y² is H and A is C=O or N-R², wherein R² is H, methyl or CH₂CH₂N(CH₃)₂ and X¹ is H or Cl. In particular in formulas I and/or II R¹ is H, methyl, ethyl or alkali metal, Y¹ is F, Y² is H and A is N-R², wherein R² is H, methyl or CH₂CH₂N(CH₃)₂ and X¹ is H or Cl and X¹ is attached to C-11 in formula I or X¹ is attached to C-9 in formula II. Most preferred are quinoxalines of formulas I and/or II wherein R¹ is H, methyl, ethyl or metal, alkali metal, such as Na⁺ or Zn²⁺, Y¹ is F and A is C=O and X¹ is H.

Also favoured are quinoxalines of formulas I and/or II in which R¹ is H, methyl, ethyl or an alkali metal cation, Y¹ is F, Y² is H and A is C(R³)₂, wherein each R³ independently is H, methyl or CH₂CH₂N(CH₃)₂ and X¹ is H or Cl. In particular in formulas I and/or II R¹ is H, methyl, ethyl or alkali metal cation, Y¹ is F, Y² is H and A is C(R³)₂, wherein each R³ independently is H, methyl or CH₂CH₂N(CH₃)₂ and X¹ is H or Cl and X¹ is attached to C-11 in formula I or X¹ is attached to C-9 in formula II. Most preferred are quinoxalines of formulas I and/or II and R¹ is H, methyl, ethyl or metal, alkali metal, such as Na⁺ or Zn²⁺, Y¹ is F, Y² is H and A is C(R³)₂, wherein both R³ are H and methyl, or H and CH₂CH₂N(CH₃)₂, and X¹ is H.

A favoured quinoxaline of formulas I and/or II is selected from ethyl-11-chloro-1-cyclopropyl-6-fluoro-4-oxo-4,8-dihydro-1H-indolo[3,2-b]pyrido [2,3-f]quinoxaline-3-carboxylate, ethyl-1-cyclopropyl-6-fluoro-4-oxo-4,8-dihydro-1H-indolo[3,2-b]pyrido[2,3-f]quinoxaline-3-carboxylate, ethyl 1-cyclopropyl-6-fluoro-12-methyl-4-oxo-4,12-dihydro-1H-indolo[2,3-b]pyrido [2,3-f]quinoxaline-3-carboxylate, ethyl- 1-cyclopropyl-6-fluoro-8-methyl-4-oxo-4,8-dihydro-1H-indolo[3,2-b]pyrido[2,3-f] quinoxaline-3-carboxylate, 1-cyclopropyl-6-fluoro-8-methyl-4-oxo-4,8-dihydro-1H-indolo [2,3-b]pyrido[2,3-f]quinoxaline-3-carboxylic acid, 1-cyclopropyl-6-fluoro-4-oxo-4,8-dihydro-1H-indolo[2,3-b]pyrido[2,3-f]quinoxaline-3-carboxy acid, 11-chloro-1-cyclopropyl-6-fluoro-4-oxo-4,8-dihydro-1H-indolo[2,3-b]pyrido [2,3-f]quinoxaline-3-carboxy acid, ethyl 1-cyclopropyl-6-fluoro-4,8-dioxo-4,8-dihydro-1H-indeno[2,1-b]pyrido[2,3-f] quinoxaline-3-carboxylate, ethyl 1-cyclopropyl-6-fluoro-4,12-dioxo-4,12-dihydro-1H-indeno[1,2-b]pyrido[2,3-f] quinoxaline-3-carboxylate, 1-cyclopropyl-6-fluoro-4,8-dioxo-4,8-dihydro-1H-indeno[2,1-b]pyrido[2,3-f] quinoxaline-3-carboxylic acid and/or 1-cyclopropyl-6-fluoro-4,12-dioxo-4,12-dihydro-1H-indeno[1, 2-b]pyrido[2,3-f] quinoxaline-3-carboxylic acid. Or 1-cyclopropyl-5-fluoro-8-methyl-4-oxo-4,8-dihydro-1H-indolo [2,3-b]pyrido[2,3-f]quinoxaline-3-carboxylic acid or ester, 1-cyclopropyl-5,6-difluoro-4-oxo-4,8-dihydro-1H-indolo[2,3-b]pyrido[2,3-f]quinoxaline-3-carboxy acid or ester, 11-chloro-1-cyclopropyl-5-fluoro-4-oxo-4,8-dihydro-1H-indolo[2,3-b]pyrido [2,3-f]quinoxaline-3-carboxy acid or ester, 1-cyclopropyl-5-fluoro-4,8-dioxo-4,8-dihydro-1H-indeno[2,1-b]pyrido[2,3-f] quinoxaline-3-carboxy acid or ester, 1-cyclopropyl-5,6-difluoro-4,12-dioxo-4,12-dihydro-1H-indeno[1,2-b]pyrido[2,3-f] quinoxaline-3-carboxy acid or ester, 1-cyclopropyl-6-fluoro-4,12-dioxo-4,12-dihydro-1H-indeno[1,2-*b*]-and 1-cyclopropyl-6-fluoro-4,12-dioxo-4,12-dihydro-1H-indeno[2,1-*b*]pyrido [2,3-*f*]quinoxaline-3-carboxylic acids and esters, such as methyl or ethyl esters.

Object of the present invention is a synthesis for preparing quinoxalines represented by general formulas I and/or II, 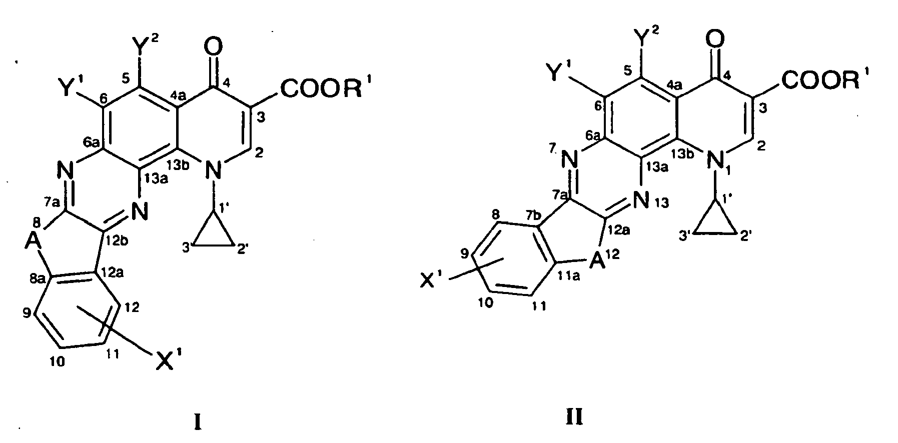
- wherein the COOR¹ group independently is an acid, an ester and/or a salt, in particular R¹ independently is H, a linear, branched or cyclic unsubstituted or substituted hydrocarbon, for example a linear or branched C₁-C₆-alkyl, in particular methyl, ethyl, iso-propyl, n-propyl, butyl, n-butyl, iso-butyl, tert-butyl, an alkali and/or alkaline earth metal, as cation, as defined above;
- Y¹ and/or Y² are independently halogen and/or H, in particular F, Cl, Br or I, especially F, most preferred Y¹ is F and Y² is H, Y¹ is H and Y² is F or Y¹ and Y² are both fluorine;
- A is oxo (C=O), C(R³)₂ and/or N-R², wherein each R³ and/or R² independently are H, linear, branched or cyclic C₁-C₆-alkyl or C₁-C₁₂-alkylamin and
- X¹ is H, a linear, branched or cyclic unsubstituted or substituted hydrocarbon or halogen, in particular F, Cl, Br or J;
- wherein a key precursor of general formula IV and wherein Y¹, Y² and R¹ are defined as above is reacted 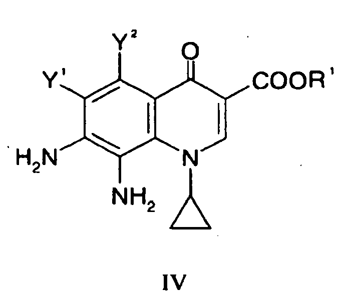
- with a substance of general formula III, such as an indane, methyl-istatine,
   istatine, indane-1,2-dion or an halogenated or alkylated derivative, 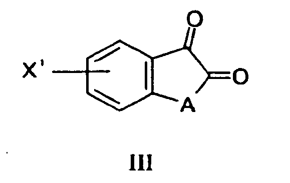
- wherein A and X¹ are as defined above, optional in presence of an acid.

In particular the precursor of formula IV is reacted with isatin, methyl-isatin, indane-1,2,3-trion, indane-1,2-dion or an halogenated or alkylated derivative, wherein X¹ is H, halogen, in particular F, Cl, Br or J, especially X¹ is H, Cl or F, if necessary in presence of an acid.

In general a key precursor of general formula IV is reacted with a substance of general formula III in presence of an acid, wherein the acid comprises a mineral acid or an organic acid. These acids are known to the skilled person as an example the acid can be selected from HCl, HBr, HJ, H₂SO₄, sulfonic acid, H₃PO₄, polyphosphonic acid, acetic acid, formic acid or another acid - as concentrated or diluted solution - that can be used for the reaction of a diamine with a dion. The preferred acid is polyphosphoric acid.

A further object of the present invention is a key precursor of general formula IV, in particular for the synthesis of quinoxalines of general formulas I and/or II, 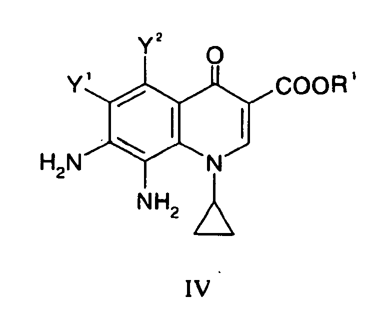
- wherein the COOR¹ group forms independently an acid, an ester and/or a salt, in particular R¹ is independently H, a linear, branched or cyclic unsubstituted or substituted hydrocarbon, for example a linear or branched C₁-C₆-alkyl, in particular methyl, ethyl, iso-propyl, n-propyl, butyl, n-butyl, iso-butyl, tert-butyl, an alkali and/or alkaline earth metal as defined above,
- Y¹ and/or Y² are independently halogen and/or H, in particular F, Cl, Br or I, especially F, most preferred Y¹ is F and Y² is H, Y¹ is H and Y² is F or Y¹ and Y² are both fluorine; and/or
- a polymorph or a solvate of the precursor.

For preferred key precursors R¹ is H, Y¹ is fluorine and Y² is H; R¹ is methyl, Y¹ is fluorine and Y² is H; R¹ is ethyl, Y¹ is fluorine and Y² is H; R¹ is Na⁺ or Ca²⁺, Y¹ is fluorine and Y² is H; R¹ is propyl, Y¹ is fluorine and Y² is H; or R¹ is butyl, Y¹ is fluorine and Y² is H.

In addition, an object of the invention is a synthesis for preparing the key precursor of general formula IV by reduction of a substance of general formula V 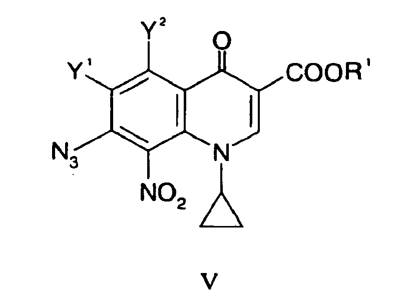
- wherein COOR¹ forms independently an acid, an ester and/or a salt, in particular R¹ is H, a linear, branched or cyclic unsubstituted or substituted hydrocarbon, for example a linear or branched C₁-C₆-alkyl, in particular methyl, ethyl, iso-propyl, n-propyl, butyl, n-butyl, iso-butyl, tert-butyl, an alkali and/or an alkali earth metal, for example Na⁺, K⁺, Li⁺, Ca²⁺, Mg²⁺,
- Y¹ and/or Y² are independently halogen and/or H, in particular F, Cl, Br or I, especially F, most preferred Y¹ is F and Y² is H, Y¹ is H and Y² is F or Y¹ and Y² are both fluorine; and/or
- wherein in particular SNCl₂ and HCl are used for the reduction of a substance of general formula V.

A further object of the present invention is the use of a quinoxaline of general formulas I and/or II as pharmaceutical active ingredient or as animal feed additive. In particular the quinoxaline of general formulas I and/or II is active as an agent having effect against cancer, as antibiotic and/or as antiviral agent.

In addition an object of the present invention is a pharmaceutical, food, animal feed or cosmetic formulation comprising quinoxaline derivatives of general formula I and/or II and at least one pharmaceutical, food, animal feed and/or cosmetic excipient. In particular the formulation against viral infections may be in form of a cream, a gel or a transdermal system.

This excipient may be selected from disintegrants, fatty acids, celluloses, such as HPMC, lactose, TiO₂, SiO₂, or other excipients the skilled person would choose.

The present invention relates further to condensation processes of indolo [2,3-*b*]quinoxalines with 4-pyridone-3-carboxylate, to give quinoxalines of general formula I and/or II, exemplified by **5 / 6** and **9 / 10** as depicted in Schemes 1 and 2. These hybrid pentacyclic heterocycles are bioactive arising from the combination of bioactive entities of which the 4-pyridone moiety (ring A in **5, 6, 9, 10**) forms an integral part of the fluoroquinolone antibacterial agents.

Cyclocondensation reaction of ethyl 7,8-diamino-1-cyclopropyl-6-fluoro-4-oxo-1,4-dihydroquinoline-3-carboxylate with 1-methylisatin produced a separable mixture of the corresponding indolo[3,2-*b*]- and [2,3-*b*]pyrido[2,3-*f*]quinoxaline-3-carboxylates, of which the latter isomer predominates. On the other hand, interaction with 1*H*-isatin or 5-chloroisatin gave the respective indolo[2,3-*b*]pyrido[2,3-*f*]quinoxaline-3-carboxylates as the sole regiospecific products that were isolated. The structures of these new pentacyclic derivatives are based on microanalytical, spectral (IR, MS, and NMR) and X-Ray crystal structure data.

### General Synthesis

The cyclocondensation reaction of ethyl 7,8-diamino-1-cyclopropyl-6-fluoro-4-oxo-1,4-dihydroquinoline-3carboxylate (**4**, relating to general formula IV) with 1-methylisatin (3, relating to general formula III), induced by polyphosphoric acid (*PPA*), is investigated. This process, involves two consecutive condensations between the unsym-diamine **4** and the *unsym*-dione **3**, and is likely to produce two isomeric products (**5, 6**, relating to general formulas I and/or II), depending on which amino group of **4** initiates the reaction *via* nucleophilic addition at the keto group in 3 (Scheme 1). As would be expected, initial addition of the more nucleophilic amino group (residing at C-8) occurs onto the more electrophilic carbon-3 of the keto group with consequent formation of ethyl 1-cyclopropyl-6-fluoro-8-methyl-4-oxo-4,8-dihydro-1*H*-indolo[2,3-*b*]pyrido[2,3-*f*]quinoxaline-3-carboxylate (**5**) as the major regioselective product in 58% yield. Conversely, initiation of the reaction by the less nucleophilic amino group (appended at C-7) takes place to a much lesser extent to form ethyl 1-cyclopropyl-6-fluoro-12-methyl-4-oxo-4,12-dihydro-1*H*-indolo[3,2-*b*]pyrido[2,3-*f*]quinoxaline-3-carboxylate (**6**) in about 2% yield. The isomeric products (**5**, **6**) were separated from the reaction mixture on preparative silica gel TLC plates.

A methyl-isatine derivative with X¹ = H can be prepared from isatin via direct N-alkylation with dimethylsulfate if necessary in presence of alkaline (NaOHaq). For example isatine is reacted with dimethylsulfate ((CH₃)₂SO₄) an 10 (w/w) weigth-% NaOH for about 2 h at 25 °C. The crude product may be purified by several crystallization steps from 25 (w/w) weight-% ethanol.

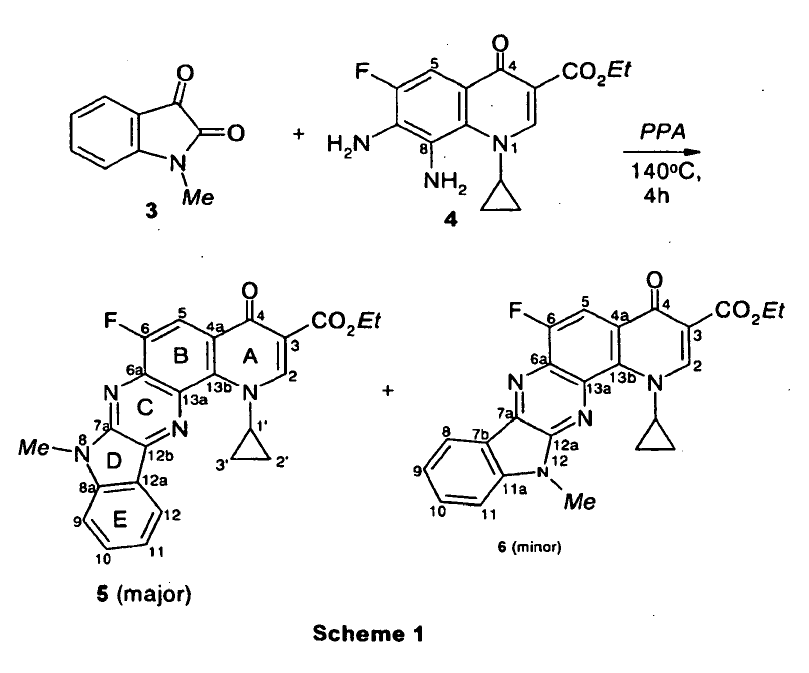

Under similar reaction conditions, cyclocondensation of **4** with 1*H*-isatins (**7, 8**) proceeded in a regiospecific manner and gave the corresponding indolo[2,3-*b*]quinoxaline-3-carboxylates (**9, 10**) as the sole products, whilst the respective isomeric indolo[3,2-*b*]quinoxaline-3-carboxylates (**11, 12**) could not be isolated and were not detected respectively under theses specific conditions (Scheme 2). More reactive reaction conditions furnish a certain, isolable amount of **11** and/or **12**. Indane-1,2-dion derivatives can be condensed via an analogous reaction with the key precursor of general formula IV to give quinoxaline derivatives of general formulas I and/or II.

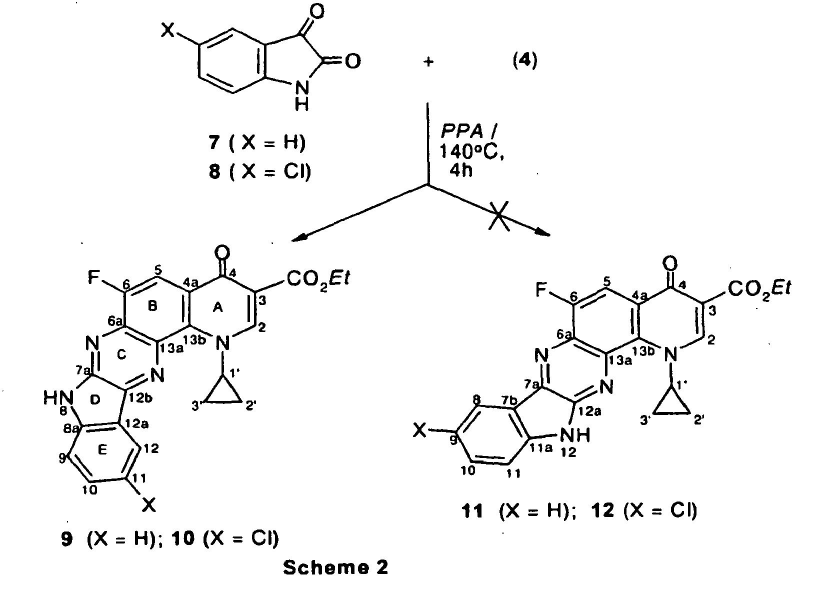

The structure of **9** (and by inference **10**) is unequivocally established via methylation of its indolic NH group; the resulting N-methylated product was shown to be identical (IR and NMR data) with an authentic sample of **5** (accessible from N-methylisatin and **4** / Scheme 1) the structure of which is confirmed by X-ray data (*vide infra*).

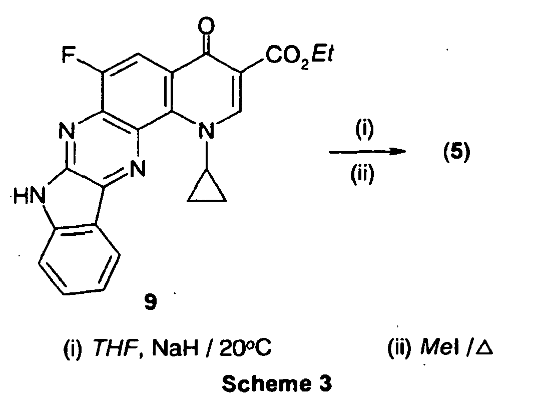

### Spectroscopic data

The IR, MS, NMR spectral data and microanalyses for the new compounds **5, 6, 9,** and **10** are in accordance with the assigned structures; details are given in the experimental part. Thus, the mass spectra display the correct molecular ion peaks for which the measured high resolution (HRMS) data are in good agreement with the calculated values. DEPT and 2D (COSY, HMQC, HMBC) experiments showed correlations that helped in the ¹H- and ¹³C-signal assignments to the different carbons and the attached / neighboring hydrogens in compounds **5, 6, 9**, and **10**. In the ¹³C-NMR spectrum of **5**, carbon-7a resonates as a doublet centered at 144.5 ppm due to spin-spin coupling with the fluorine atom (⁴*J*_{C-F} = 1.5 Hz), while the more distant carbon-12b appears as a singlet at δ 137.1 ppm. Long-range correlations are observed between H-12 and each of C-12b and C-8a, as well as between the N(8)-C*H*₃ protons and the C-7a doublet. These ¹H - ¹³C correlations are compatible with the assigned structure for 5 which is further confirmed by X-ray crystallography (*vide infra*). On the other hand, the HMBC experiment for **6** shows that the N(12)-C*H*₃ protons are strongly correlated with the C-12a singlet at 143.2 ppm (as well as with the C-11 a signal at 145.4 ppm), while the C-7a doublet at 139.5 (⁴*J*_{C-F} = 1.7 Hz) is correlated with H-8. These three-bond correlations are in conformity with the suggested structure for **6**.
Corresponding long-range correlations are also observed in the HMBC experiments for **9** and **10**. Thus, H-12 is correlated with each of C-8a, C-12b and C-10, H-10 with C-8a, while H-9 is correlated with C-12a and C-11.

### X-Ray Structure

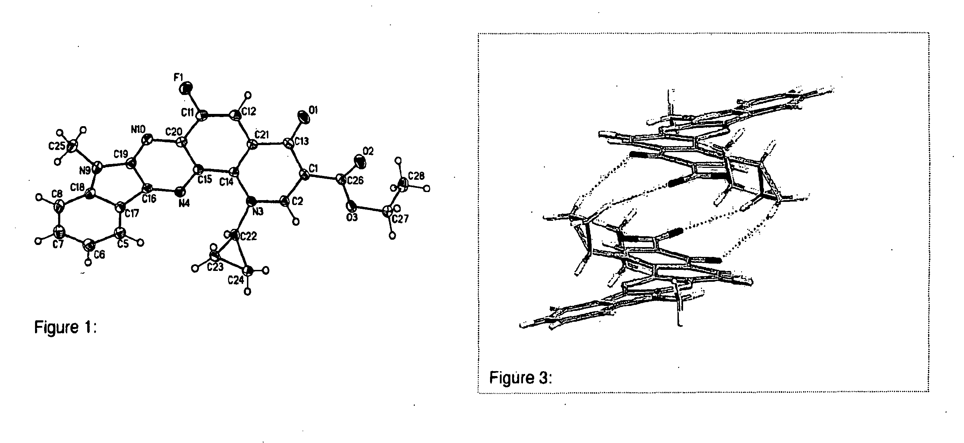

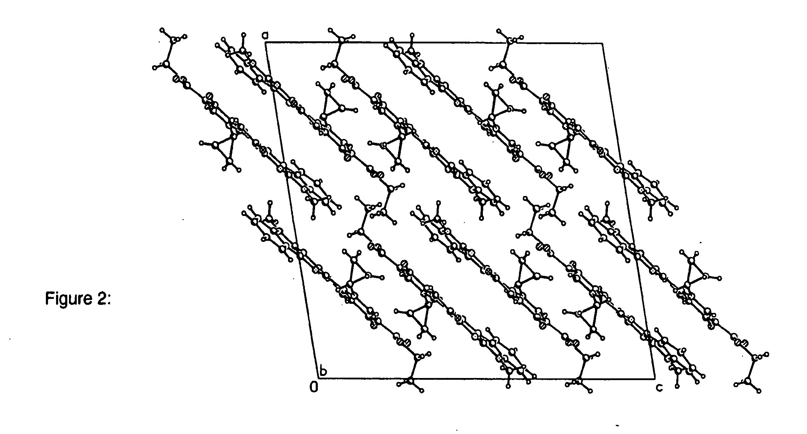

An X-Ray crystal structure determination was performed to confirm the structure of **5.** A summary of data collection and refinement parameters is given in Table 1. The molecular structure of **5,** based on crystallographic data, is displayed in Fig. 2, while Fig. 3 shows a packing diagram for **5.** The molecule adopts an almost planar configuration with the cyclopropyl ring in a likewise expected conformation. The intermolecular interactions of pairwise grouped molecules around inversion centers in the solid state are dominated by C-H···O weak hydrogen bonds between the *syn*-cyclopropyl H-atoms and the carbonyl groups, with distances of 2.51 and 2.49 Å, respectively for H24A···O1 and H23B···O2, and C-H···O bond angles 138.3° and 160.6° as displayed in Fig. 2.

**Table 1. Summary of the crystal data and structure refinement parameters for 5**

| | |
|---|---|
| Empirical formula | C₂₄ H₁₉ F N₄ O₃ |
| Formula weight | 430.43 Da |
| Temperature / K | 203(2) |
| Wavelength /Å | 0.71073 |
| Crystal system | monoclinic |
| Space group | / 2/*a* |
| Unit cell dimensions | |
| *a* /Å | 20.4734(12) |
| *b* /Å | 9.0443(5) |
| *c* /Å | 21.3106(19) |
| β/°/ | 99.828(2) |
| Volume /Å³ | 3888.1 (5) |
| Z | 8 |
| Calculated density /g cm⁻³ | 1.471 |
| Absorption coefficient / mm⁻¹ | 0.106 |
| *F* (000) | 1792 |
| Theta range for data collection/° | 1.94 - 32.17 |
| completeness to theta = 32.17° | 97.8 % |
| Index range | -26 ≤ *h* ≤ 30; -13 ≤ *k* ≤ 12 ; -31 ≤ *1* ≤ 25 |
| Reflections collected | 45127 |
| Independent reflections | 6709 [ *R ᵢₙₜ* = 0.0295] |
| Weight scheme | Calcd *w* = 1 [σ² (*F₀*)² + (0.0707*P*)² + 1.1917*P*] where *P* = [(*F₀*)² + 2(*F_{c}*)²] / 3 |
| Data / restraints / parameters | 5145 / 0 / 290 |
| Goodness-of-fit on *F*² | 1.042 |
| Final *R* indices [ *I* > 2σ ( *I* )] | *R*₁ = 0.0465, *wR*₂ = 0.1211 |
| *R* indices ( all data) | *R*₁ = 0.0641, *wR*₂ = 0.1324 |
| Largest difference peak /e. Å⁻³ | 0.386 |
| Largest difference hole /e. Å⁻³ | -0.276 |

### Examples:

### Compounds 5a/b, 6a/b, 9a/b and 10a/B

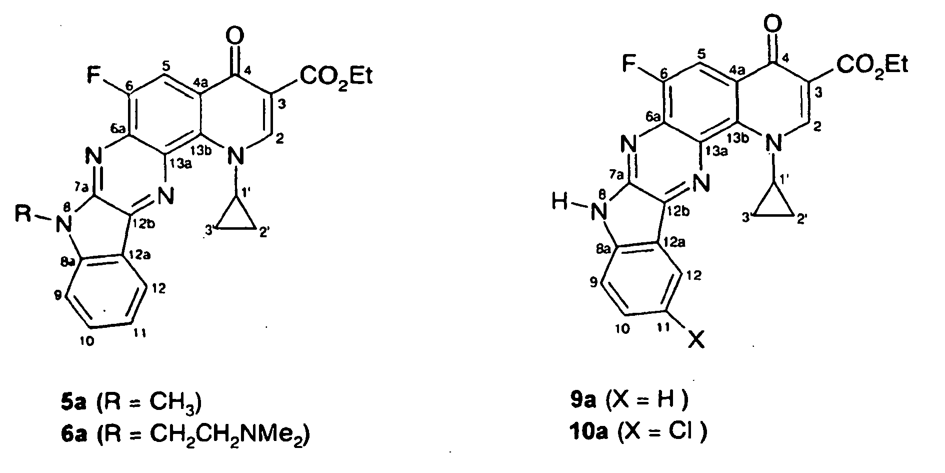

Data for compounds **5a /9a /10a** are given below. Compound **6a** can be prepared in the same manner or according to a synthesis known to the skilled person.

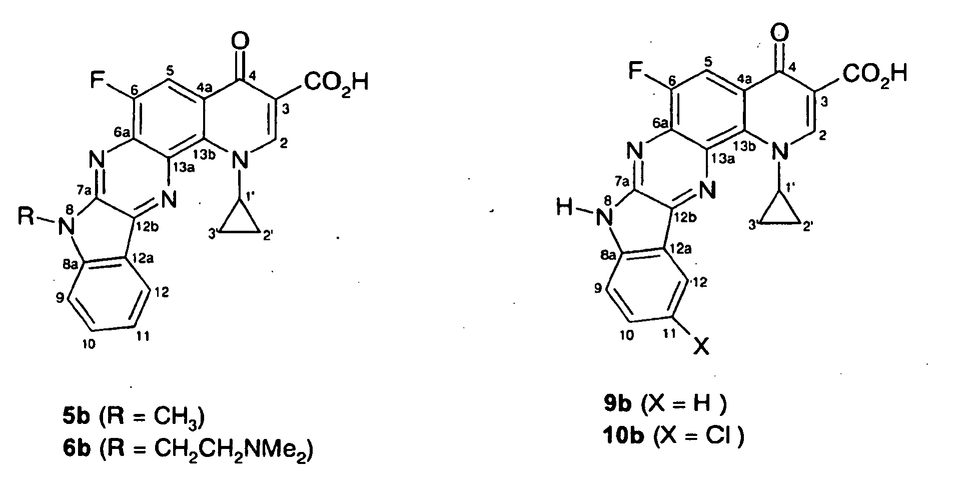

Compounds **5b, 6b, 9b** and **10b** can be prepared in essentially the same manner as described below.

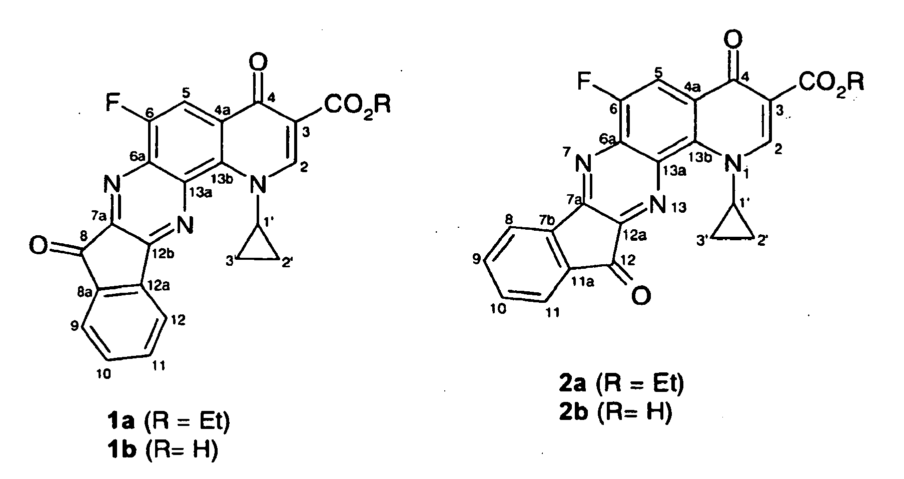

Names and data for compounds **1a / 1b / 2a / 2b** are given below. These derivatives are potent antitumor agents.

Compounds **13b, 14b, 15b** and/or **16b** as well as their analogous esters, for example as methyl or ethyl esters, can be prepared according to the present disclosed process.

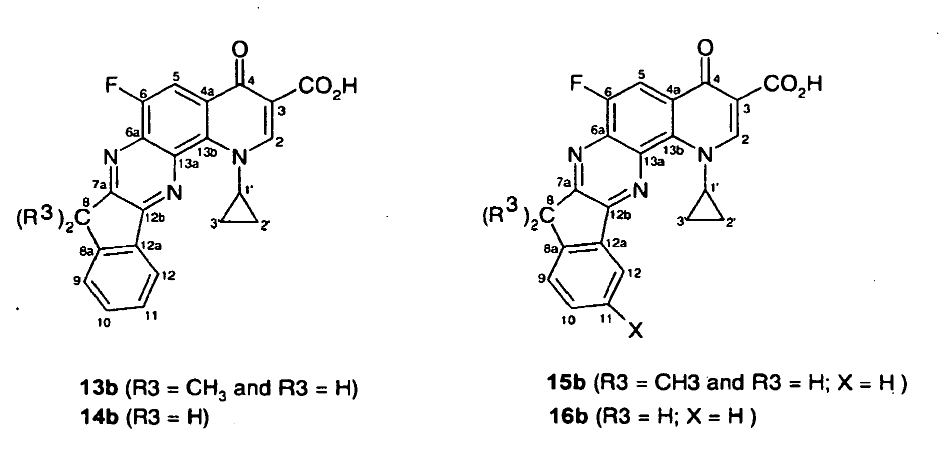

### Experimental

2,4-Dichloro-5-fluoro-3-nitrobenzoic acid, ethyl 3-(*N*,*N*-dimethylamino)acrylate, cyclopropyl-amine, isatin, 5-chloroisatin, 1-methylisatin and indane-1,2,3-trione were purchased from Acros. Melting points were determined on a Gallenkamp electrothermal melting-temperature apparatus. IR spectra were recorded as KBr discs on a Nicolet Impact-400 FT-IR spectrophotometer. ¹H- and ¹³C NMR spectra were measured on a Bruker DPX-300 instrument. Chemical shifts are expressed in ppm with reference to *TMS* as internal standard. High resolution mass spectra (HRMS) were measured in positive ion mode by Electrospray (ESI) on APEX-Qe 94 instrument. The samples were dissolved in acetonitrile, diluted in spray solution (methanol/water 1:1 *vlv* + 0.1 % formic acid) and infused using a syringe pump with a flow of 2 mm³/min. External calibration was conducted using Arginine cluster in a mass range *m*/*z* 175-871. Elemental analyses (C, H, N, Cl) were preformed at the Microanalytical Laboratory of the Hashemite University, Zarqa-Jordan, and the results were found to be in good agreement (± 0.4%) with the calculated values. (if not otherwise indicated)

### Example 1

**Ethyl-7,8-diamino-1-cyclopropyl-6-fluoro-4-oxo-1,4-dihydroquinoline-3-carboxylate** (formula IV) is prepared by reduction of ethyl 7-azido-1-cyclopropyl-6-flouro-8-nitro-4-oxo-1,4-dihydroquinoline-3-carboxylate (Al-Hiari YM, Khanfar MA, Qaisi AM, Abu Shuheil MY, El- Abadelah MM, Boese R (2006) Heterocycles **68**: 1163, and references cited therein) using SnCl₂ and conc HCl at room temp according to the following procedure described in Scheme 3 or an analogous one and detailed in the Experimental part. High resolution MS data and ¹³C-NMR signal assignments (based on DEPT and 2D: COSY, HMQC and HMBC experiments) of intermediates **5 - 7** of Scheme **3,** are in conformity with the assigned structures and are given herewith.

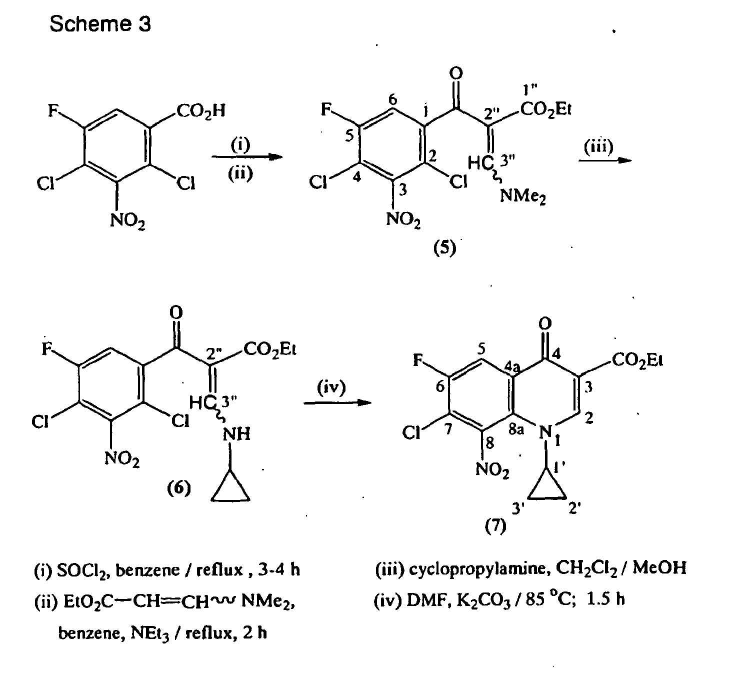

The structure of **3** is evidenced from its spectroscopic data and confirmed by single X-ray crystal structure determination (*vide infra*). The intermediate, 7-azido-8-nitroquinolone (**8** of **Scheme 4**), is obtained *via* direct interaction of sodium azide with ethyl 7-chloro-1-cyclopropyl-6-fluoro-8-nitro-4-oxo-1,4-dihydroquinoline-3-carboxylate (**7**) in dimethylsulfoxide at rt.

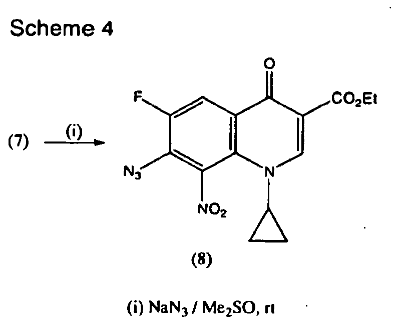

Analogous intermediates with halogenated C-6 and/or C-5 atoms can be obtained via an analogous process, as for example ethyl 7-chloro-1-cyclopropyl-5-fluoro-8-nitro-4-oxo-1,4-dihydroquinoline-3-carboxylate, ethyl 7-chloro-1-cyclopropyl-5,6-difluoro-8-nitro-4-oxo-1,4-dihydroquinoline-3-carboxylate, ethyl 7-chloro-1-cyclopropyl-6-chloro-8-nitro-4-oxo-1,4-dihydroquinoline-3-carboxylate, ethyl 7-chloro-1-cyclopropyl-5,6-dichloro-8-nftro-4-oxo-1,4-dihydroquinoline-3-carboxylate or ethyl 7-chloro-1-cyclopropyt-5-chloro-8-nitro-4-oxo-1,4-dihydroquinoline-3-carboxylate that can be reduced to ethyl-7,8-diamino-1-cyclopropyl-5,6-(Y1,Y2 substituted)-4-oxo-1,4-dihydroquinoline-3-carboxylates of general formula IV. For the reduction to the diamine of general formula IV the skilled person knows different applicable reduction media and processes.

### EXPERIMENTAL relating to Example 1

2,4-Dichloro-5-fluoro-3-nitrobenzoic acid, ethyl 3-(dimethylamino)acrylate and cyclopropylamine were purchased from Acros. Melting points (uncorrected) were determined on a Gallenkamp electrothermal melting temperature apparatus. ¹Hand ¹³C-NMR spectra were measured on a Bruker DPX-300 instrument with Me₃Si as internal reference. EIMS spectra were obtained using a Finnigan MAT TSQ-70 spectrometer at 70 eV; ion source temperature = 200 °C. High resolution MS-ESI data were obtained with Bruker Bio TOF III. IR spectra were recorded as KBr discs on a Nicolet Impact-400 FT-IR spectrophotometer. Microanalyses were preformed at the Microanalytical Laboratory - Medicinal Chemistry division, Faculty of Pharmacy, Jordan University, Amman, Jordan.

### Example 1.1a

### Ethyl 3-(N,N-dimethylamino)-2-(2,4-dichloro-5-fluoro-3-nitrobenzoyl) acrylate (5 of Scheme 3)

A mixture of 2,4-dichloro-5-fluoro-3-nitrobenzoic acid (10.2 g, 40 mmol) and thionyl chloride (19.0 g, 160 mmol) in dry benzene (120 ml) was refluxed for 3 - 4 h under anhydrous conditions. The solvent and excess thionyl chloride were then distilled off under reduced pressure, and dry benzene (20 ml) was then introduced into the reaction vessel and re-distilled so as to remove traces of thionyl chloride. The resulting 2,4-dichloro-5-fluoro-3-nitrobenzoyl chloride, formed as thick oil, was used as such for the next step without further purification.
To a stirred and cooled (5-10 °C) solution of ethyl 3-(*N,N*-dimethylamino)acrylate (6.3 g, 44 mmol) and triethylamine (8.1 g, 80 mmol) in dry benzene (50 ml) was added dropwise a solution of the crude acid chloride (prepared above) in dry benzene (25 ml). The resulting mixture was refluxed for 2 h, then cooled to rt and washed with water (2 x 30 ml). The organic layer was separated, dried (anhydrous MgSO₄) and the solvent benzene was then evaporated to dryness under reduced pressure. The residual product was soaked in methanol (10 ml) whereby the title compound **5** was produced as yellowish powder which was collected by suction filtration and dried. Yield = 13.8 g (91%).
IR (KBr): *v* 3074, 3037, 2988, 2928, 2873, 1689, 1619, 1554, 1454, 1397, 1375, 1344, 1321, 1278, 1206, 1177, 1129, 1030 cm⁻¹; ¹H NMR (300 MHz, CDCl₃): δ 0.95 (t, *J* = 7.1 Hz, 3H, C*H*₃), 2.97 (s, 3H) and 3.37 (s, 3H) [*N* (CH₃)₂], 3.94 (q, *J* = 7.1 Hz, 2H, C*H*₂Me), 7.27 (d, ³*J*_{H-F} = 8.2 Hz, 1H, H-6), 7.91 (br s, 1H, *N*-C(3")-H); ¹³C NMR (75 MHz, CDCl₃): δ 13.8 (CH₃CH₂), 43.3, 48.4 [*N* (CH₃)₂], 60.2 (CH₂Me), 100.9 (C-2"), 114.5 (d, ²*J*_{C-F} = 23.3 Hz, C-4), 116.9 (d, ²*J*_{C-F} = 23.1 Hz, C-6), 118.2 (d, ³*J*_{C-F} = 4.5 Hz, C-1), 144.2 (d, ³*J*_{C-F} = 6 Hz, C-3), 148.8 (br d, ⁴*J*_{C-F} = 1.3 Hz, C-2), 156.6 (d, ¹*J*_{C-F} = 254 Hz, C-5), 160.5 (*N*-C-3"), 166.5 (CO₂Et), 185.1 (C = O).

### Example 1.1b

### Ethyl 3-(N-cyclopropylamino)-2-(2,4-dichloro-5-fluoro-3-nitrobenzoyl) acrylate (6 of Scheme 3)

A stirred solution of 5 obtainable from example 1.1a (14.4 g, 38 mmol) in dichloromethane (50 ml) and methanol (10 ml), cooled to 8 - 10 °C, was treated dropwise with cyclopropylamine (3.2 g, 56 mmol) in MeOH (3 ml), and the resulting mixture was further stirred for additional 10 - 15 min at 8 - 10 °C. Methanol (90 ml) was then added and the reaction mixture was stirred at rt for 1 - 2 h. The precipitated white solid product was filtered, washed with cold ethanol (95%, 10 ml) and dried. Yield 11.0 g; a second crop of 6 (1.7g) was obtained upon concentration of the mother liquor. Total yield of 6 = 13.8 g (93%). *Anal*.Calcd for C₁₅H₁₃Cl₂FN₂O₅ (390.18): C, 46.06; H, 3.35; Cl, 18.13; N, 7.16; found C,00.00; H,0.00; Cl, 00.00; N,00.00; IR (KBr): *v* 3228, 3196, 3157, 3032, 2984, 2906, 1679,1618,1561,1545,1455, 1402, 1369, 1342, 1316, 1253, 1192, 1151, 1113, 1068, 1021 cm⁻¹;
¹H NMR (300 MHz, CDCl₃): δ 0.88 (m, 4H, H₂-2' / H₂-3'), 1.01, 0.92 (*Z* /*E*, 2t, *J* = 7.1 Hz, 3H, C*H*₃), 3.00 (m, 1H, H-1'), 3.99, 3.73 (*Z* /*E*, 2q, *J* = 7.1 Hz, 2H, C*H*₂Me), 7.10, 7.16 (*Z* /*E*, 2d, ³*J*_{H-F} = 8.1 Hz, 1H, H-6), 8.26, 8.35 (*Z* /*E*, 2d, *J* = 14 Hz, 1H, *N*-C(3")-H), 11.01, 9.77 (Z /*E*, 2 br d, *J* = 14 Hz, 1H, exchangeable N-H); ¹³C NMR (75 MHz, CDCl₃): δ 6.7 (C-2'/ C-3' ), 13.9, 13.3 (*Z* /*E*, *C*H₃), 31.0, 30.3 (*Z* /*E*, C-1' ), 60.2, 60.1 (*Z* /*E*, CH₂Me), 100.3, 99.8 (*Z* /*E*, C-2"), 114.1, 114.0 (*Z* /*E*, 2d, ²*J*_{C-F} = 23.3 Hz, C-4), 115.7, 116.0 (*Z* /*E*, 2d, ²*J*_{C-F} = 23.3 Hz, C-6), 117.7, 117.9 (*Z* /*E*, 2d, ³*J*_{C-F} = 4.7 Hz, C-1), 143.9, 143.7 (*Z* /*E*, 2d, ³*J*_{C-F} = 6.2 Hz, C-3), 148.6 (br d, ⁴*J*_{C-F} = 1.4 Hz, C-2), 156.7 (d, ¹*J*_{C-F} = 254 Hz, C-5), 161.9, 161.4 (*Z* /*E*, N-C-3"), 165.7, 167.8 (*Z* /*E*, *C*O₂Et), 188.4, 186.2 (*Z* /*E*, C = O).

### Example 1.1c

### Ethyl 7-chloro-1-cyclopropyl-6-fluoro-8-nitro-4-oxo-1,4-dihydroquinoline-3-carboxylate (7 of Scheme 3)

A solution of 6 obtainable from example 1.1.b (11.7 g, 30 mmol) in DMF (50 ml) and potassium carbonate (12.4 g, 90 mmol) was heated at 85 °C. The progress of the cyclization reaction was monitored by tlc (eluent: AcOEt + n-hexane, 1:1 v/v) and was completed within 80 to 90 min. The reaction mixture was then poured slowly onto crushed ice (500 g) under vigorous stirring, the precipitated pale yellow solid product was collected, washed with water, triturated with cold ethanol and dried. Yield of **7** = 9.5 g (89%).
Anal.Calcd for C₁₅H₁₂ClFN₂O₅ (354.72): C, 50.79; H, 3.41; Cl, 9.99; N, 7.90; found C.50.96; H,3.35; Cl, 9.83; N,7.71;
¹H NMR (300 MHz, CDCl₃): δ 1.11 (m, 4H, H₂-2' / H₂-3'), 1.33 (t, *J* = 7.1 Hz, 3H, C*H*₃CH*₂*), 3.57 (m, 1H, H-1'), 4.30 (q, *J* = 7.1 Hz, 2H, *CH*₂Me), 8.27 (d, ³*J*_{H-F} = 8.0 Hz. 1H, H-5), 8.56 (s, 1H, H-2); ¹³C NMR (75 MHz, CDCl₃): δ.11.1 (C-2'/ C-3' ). **14.4** (CH₃CH₂), 37.9 (C-1' ), 61.4 (CH₂Me), 111.8 (C-3), 115.6 (d, ²*J*_{C-F} = 23.0 Hz, C-5), 122.0 (d, ²*J*_{C-F} = 23.7 Hz, C-7), 130.1 (d, ³*J*_{C-F} = 5.0 Hz, C-4a), 130.8 (d, ⁴*J*_{C-F} = 3.0 Hz, C-8a), 140.8 (d, ³*J*_{C-F} = 1.6 Hz, C-8), 151.8 (C-2), 154.4 (d, ¹*J*_{C-F} = 258 Hz, C-6), 164.0 (CO₂Et), 170.8 (d, ⁴*J*_{C-F} = 2 Hz, C-4).

### Example 1.1d

### Ethyl 7-azido-1-cyclopropyl-6-fluoro-8-nitro-4-oxo-1,4-dihydroquinoline-3-carboxylate (8 of Scheme 4)

Sodium azide (1.5 g, 23 mmol) was added to a solution of **7** obtainable from example **1.1.c** (7.1 g, 20 mmol) in dimethylsulfoxide (50 ml). The resulting mixture acquired white turbidity within few minutes, and was further stirred at room temperature for 1 - 2 h. Thereafter, the reaction mixture was diluted with cold water (250 ml), and the precipitated solid product was collected under suction, washed with cold water and dried. Yield of **8** = 5.6 g (78 %), mp 184 - 185 °C (decomposition).

### Example 2

### Ethyl 1-cyclopropyl-6-fluoro-8-methyl-4-oxo-4,8-dihydro-1H-indolo[2,3-b]pyrido[2,3-f] quinoxaline-3-carboxylate (5a, C₂₄H₁₉FN₄O₃)

A stirred suspension of ethyl 7,8-diamino-1-cyclopropyl-6-fluoro-4-oxo-1,4-dihydro-quinoline-3-carboxylate (0.61 g, 2 mmol) and 0.32 g 1-methylisatin (2 mmol) in 30 g of polyphosphoric acid (PPA) was heated at 140°C for 4 h. After cooling to room temp, the reaction mixture was poured with stirring onto crushed ice and extracted with 3 x 30 cm³ of CH₂Cl₂. The combined organic extracts were dried (MgSO₄), concentrated under reduced pressure, and the resulting yellow product (**5a** + its isomer) were separated on silica gel TLC plates using *AcOEt* : CHCl₃ (1:4, *v*/*v*)] as the developing solvent mixture, and recrystallized from chloroform / isopropyl ether. Yield of 5a (major product): 0.5 g (58%); mp > 300 °C (darkens at 240 °C). IR: *ν̅* = 3068, 2978, 2925, 2849, 1724, 1644, 1610, 1580, 1524, 1468, 1395, 1301, 1263, 1197, 1120, 1087, 1033 cm⁻¹; ¹H NMR (300 MHz, CDCl₃): δ = 0.91, 1.29 (2 m, H₂-2' + H₂-3'), 1.44 (t, *J* = 7.1 Hz, C*H₃*CH₂O-), 3.97 (s, N(8)-CH₃), 4.43 (q, *J* = 7.1 Hz, -OC*H*₂CH₃), 4.84 (m, H-1'), 7.41 (dd, *J* = 7.5, 7.6 Hz, H-11), 7.50 (d, *J* = 8 Hz, H-9), 7.74 (dd, *J* = 7.5, 8 Hz, H-10), 8.23 (d, *J* = 7.6 Hz, H-12), 8.37 (d, ³*J*_{H-F} = 10.8 Hz, H-5), 8.78 (s, H-2) ppm; ¹³C NMR (75 MHz, CDCl₃): δ = 11.2 (C-2' + C-3'), 14.5 (CH₃CH₂O-), 27.8 (N(8)-CH₃), 42.9 (C-1'), 61.1 (-O*C*H₂*M*e), 109.1 (d, ²*J*_{C-F} = 21.3 Hz, C-5), 110.0 (C-9), 112.1 (C-3), 119.1 (C-12a), 121.9 (C-11), 122.2 (C-12), 126.5 (d, ³*J*_{C-F} = 7.1 Hz, C-4a), 131.8 (C-10), 132.1 (d, ³*J*_{C-F} = 1.7 Hz, C-13a), 134.3 (d, ²*J*_{C-F} = 14 Hz, C-6a), 135.5 (d, ⁴*J*_{C-F} = 2.3 Hz, C-13b), 137.1 (C-12b), 144.5 (d, ⁴*J*_{C-F} = 1.5 Hz, C-7a), 145.1 (C-8a), 150.1 (C-2), 154.4 (d, ¹*J*_{C-F} = 255 Hz, C-6), 165.6 (*C*O₂*Et*), 172.7 (d, ⁴*J*_{C-F} = 2 Hz, C-4) ppm; HRMS(ESI): Calcd. for C₂₄H₂₀FN₄O₃⁺ [M+H]⁺: 431.15194, found: 431.15152.

### Example 3

### 1-cyclopropyl-6-fluoro-8-methyl-4-oxo-4,8-dihydro-1H-indolo [2,3-b]pyrido [2,3-f]quinoxaline-3-carboxylic acid (5b):

This compound was prepared from **5a** (0.20 g, 0.46 mmol) by refluxing in 12 % aqueous HCl (50 ml) and ethanol (10 ml) at 80-85 °C, for 24-30 h. Then, the reaction mixture was cooled, the resulting yellow precipitate was collected, washed with water and dried. Yield 0.17 g (91 %); mp > 300 °C. IR (KBr) *v* : 3479, 3202, 3061, 1722, 1640, 1602, 1586, 1554, 1502, 1454, 1437, 1391, 1358, 1317, 1257, 1094 cm⁻¹. HRMS (ESI): calcd. for C₂₂H₁₆FN₄O₃⁺ [M+H]⁺: 403.12064, found: 403.11989. ¹H NMR (300 MHz, DMSO-d₆): δ 1.02 (m, 2H) and 1.33 (m, 2H) (H₂-2' / H₂-3'), 4.03 (s, 3H, N(8)-CH₃), 5.14 (m, 1H, H-1'), 7.52 (m, 1H, H-11), 7.89 (two overlapped br m, 2H, H-9 + H-10), 8.28 (d, 1H, ³*J*_{H-F} = 10.6 Hz, H-5), 8.56 (d, 1H, *J* = 8.1 Hz, H-12), 9.04 (s, 1H, H-2), 15.13 (s, 1H, CO₂*H*).

### Example 4

### Collection of X-ray Diffraction Data and Structure Analysis of 5

Yellow block crystals were grown by allowing a clear solution of **5** in CHCl₃ in an open vessel to stand at room temp for 3-4 days. Crystal data collection was made with a Siemens SMART CCD diffractometer [Mo-Kα-radiation, graphite monochromator] operating in the omega scan mode (0.3°). The data were reduced with the Siemens-Bruker program suite XSCANS [Bruker AXS SADABS program multiscan V2.03] and the structure was solved by the direct method using SHEUCTL PLUS programs [Sheldrick GM, SHELXTL PLUS System of computer programs for the determination of crystal structure from the X-Ray diffraction data, Vers.5.10.DOS/WIN 95/NT]. All non-hydrogen atoms were refined anisotropically by full-matrix, least squares procedure based on *F*₂ using all unique data. Hydrogen atoms were placed in calculated positions and treated as riding groups, with the 1.2 fold (1.5 fold for methyl groups) isotropic displacement parameters of the equivalent Uij of the corresponding carbon atom. Crystallographic data for the structural analysis of 5 are going to be deposited with the Cambridge Crystallographic Data Center.

### Example 5

### Ethyl 1-cyclopropyl-6-fluoro-12-methyl-4-oxo-4,12-dihydro-1H-indolo[2,3-b]pyrido [2,3-f]quinoxaline-3-carboxylate (6), C₂₄H₁₉FN₄O₃

This compound is obtained as a minor product in the preparation of 5 (*vide supra*). Yield: 0.02 g (2.4%); mp > 300°C. IR: ν̅ = 3081, 3007, 2980, 2910, 2882, 1723, 1617, 1585, 1530, 1466, 1395, 1297, 1255, 1166, 1124, 1082 cm⁻¹; ¹H NMR (300 MHz, CDCl₃): δ = 0.98, 1.27 (2 m, H₂-2' + H₂-3'), 1.44 (t, *J* = 7.1 Hz, C*H₃*CH₂O-), 3.96 (s, N(12)-CH₃), 4.44 (q, *J* = 7.1 Hz, -OC*H₂*CH₃), 4.93 (m, H-1'), 7.45 (dd, *J* = 7.7, 7.8 Hz, H-9), 7.52 (d, *J* = 8 Hz, H-11), 7.78 (dd, *J* = 7.7, 8 Hz, H-10), 8.33 (d, ³*J*_{H-F} = 10.8 Hz, H-5), 8.55 (d, *J* = 7.8 Hz, H-8), 8.85 (s, H-2) ppm; ¹³C NMR (75 MHz, CDCl₃): δ = 11.4 (C-2' + C-3'), 14.5 (*C*H₃CH₂O-), 29.8 (N(12)-CH₃), 42.6 (C-1'), 61.2 (-O*C*H₂Me), 106.4 (d, ²*J*_{C-F} = 21.8 Hz, C-5), 109.6 (C-11), 112.0 (C-3), 118.8 (C-7b), 121.9 (C-9), 123.6 (C-8), 129.1 (d, ³*J*_{C-F} = 7.6 Hz, C-4a), 132.3 (C-10), 134.2 (d, ²*J*_{C-F} = 12_{.}4 Hz, C-6a), 134.3 (d, ⁴*J*_{C-F} = 1.8 Hz, C-13b), 137.3(C-13a),139.5 (d, ⁴*J*_{C-F} = 1.7 Hz, C-7a), 143.2 (C-12a), 145.4 (C-11a), 150.1 (C-2), 155.4 (d, ¹*J*_{C-F} = 256 Hz, C-6), 165.7 (*C*O₂*Et*), 172.8 (d, ⁴*J*_{C-F} = 2.2 Hz, C-4) ppm; HRMS (ESI): Calcd. for C₂₄H₂₀FN₄O₃⁺ [M+H]⁺: 431.15194, found: 431.15128.

### Example 6

### Ethyl-1-cyclopropyl-6-fluoro-4-oxo-4,8-dihydro-1H-indolo[2,3-b]pyrido[2,3-f]quinoxaline-3-carboxylate (9a, C₂₃H₁₇FN₄O₃)

A stirred suspension of ethyl 7,8-diamino-1-cyclopropyl-6-fluoro-4-oxo-1,4-dihydro-quinoline-3-carboxylate (0.61 g, 2 mmol) and 0.30 g isatin (2 mmol) in 30 g of *PPA* was heated at 140°C for 4 h. After cooling to room temp, the reaction mixture was poured with stirring onto crushed ice, the resulting brown precipitate was collected, triturated with methanol, and recrystallized from methanol / chloroform. Yield: 0.5 g (58%); mp > 300°C. IR: ν̅ = 3415, 3235, 2926, 2860, 1727, 1691, 1617, 1528, 1467, 1394, 1325, 1299, 1116 cm⁻¹ ; ¹H NMR (300 MHz, *DMSO*-d₆): δ = 0.94, 1.25 (2 m, H₂-2' + H₂-3'), 1.29 (t, *J* = 7.1 Hz, C*H₃*CH₂-), 4.25 (q, *J* = 7.1 Hz, -C*H₂*CH₃), 4.91 (m, H-1'), 7.41 (dd, *J* = 7.5, 7.7 Hz, H-11), 7.63 (d, *J* = 7.9 Hz, H-9), 7.75 (dd, *J* = 7.5, 7.9 Hz, H-10), 8.12 (d, ³*J*_{H-F} = 10.9 Hz, H-5), 8.43 (d, *J* = 7.7 Hz, H-12), 8.73 (s, H-2), 12.54 (s, N-H) ppm; ¹³C NMR (75 MHz, *DMSO*-d₆): δ = 11.3 (C-2' + C-3'), 14.8 (-*C*H₃CH₂), 43.5 (C-1'), 60.5 (-*C*H₂Me), 108.0 (d, ²*J*_{C-F} = 20.7 Hz, C-5), 112.0 (C-3), 113.0 (C-9), 119.5 (C-12a), 122.0 (C-11), 123.2 (C-12), 125.7 (d, ³*J*_{C-F} = 6.5 Hz, C-4a), 132.4 (C-10), 132.7 (d, ⁴*J*_{C-F} = 1.8 Hz, C-13a), 134.2 (d, ²*J*_{C-F} = 14.2 Hz, C-6a), 136.2 (d, ⁴*J*_{C-F} = 2.5 Hz, C-13b), 137.8 (C-12b), 144.7 (C-8a), 145.4 (C-7a), 150.4 (C-2), 154.2 (d, ¹*J*_{C-F} = 252 Hz, C-6), 164.8 (*C*O₂*Et*), 171.8 (d, ⁴*J*_{C-F} = 2.2 Hz, C-4) ppm; HRMS (ESI): Calcd. for C₂₃H₁₈FN₄O₃⁺ [M+H]⁺: 417.13629, found: 417.13571; calcd. for C₂₃H₁₇FN₄O₃Na⁺ [M+Na]⁺ : 439.11824, found: 439.11761.

### Example 7

### Ethyl-11-chloro-1-cyclopropyl-6-fluoro-4-oxo-4,8-dihydro-1H-indolo[2,3-b]pyrido [2,3-f]quinoxaline-3-carboxylate (10a, C₂₃H₁₆ClFN₄O₃)

This compound was prepared from ethyl 7,8-diamino-1-cyclopropyl-6-fluoro-4-oxo-1,4-dihydroquinoline-3-carboxylate (0.61 g, 2 mmol) and 0.36 g 5-chloroisatin (2 mmol) by following similar procedure and experimental conditions described above for 9a. Yield: 0.48 g (53%); mp > 300°C (darkens at 280 °C). IR: ν̅ = 3300, 3183, 3101, 2976, 1724, 1684, 1612, 1524, 1467, 1429, 1376, 1329, 1273, 1251, 1181, 1083 cm⁻¹; ¹H NMR (300 MHz, *DMF*-d₇) : δ = 1.08, 1.40 (2 m, H₂-2' + H₂-3'), 1.35 (t, *J* = 7.1 Hz, C*H*₃CH₂-), 4.33 (q, *J* = 7.1 Hz, -C*H₂*CH₃), 5.15 (m, H-1'), 7.78 (dd, *J* = 8.7, 0.8 Hz, H-9), 7.82 (dd, *J* = 8.7, 1.9 Hz, H-10), 8.26 (d, ³*J*_{H-F} = 11.0 Hz, H-5), 8.62 (dd, *J* = 1.9, 0.8 Hz, H-12), 8.87 (s, H-2), 12.61 (br s, N-H) ppm; ¹³C NMR (75 MHz, *DMF*-d₇): δ = 10.9 (C-2' + C-3'), 14.2 (-CH₃CH₂), 43.3 (C-1'), 60.4 (-CH₂*Me*), 108.3 (d, ²*J*_{C-F} = 21.0 Hz, C-5), 112.9 (C-3), 114.5 (C-9), 121.2 (C-12a), 122.5 (C-12), 126.2 (d, ³*J*_{C-F} = 6.5 Hz, C-4a), 126.8 (C-11), 132.0 (d, ³*J*_{C-F} = 1.6, C-13a), 131.9 (C-10), 134.7 (d, ²*J*_{C-F} = 15.2 Hz, C-6a), 136.5 (C-13b), 136.9 (C-12b), 143.4 (C-8a), 145.8 (C-7a), 150.3 (C-2), 154.4 (d, ¹*J*_{C-F} = 253 Hz, C-6), 164.7 (CO₂*Et*), 171.9 (d, ⁴*J*_{C-F} = 2.0 Hz, C-4) ppm; HRMS (ESI): calcd. for C₂₂H₁₇ClFN₄O₃⁺ [M+H]⁺: 451.09732, found: 451.09685.

### Example 7 - Transformation of 9a into 5a

Sodium hydride 0.12 g (60% dispersion in mineral oil, 3 mmol) was added portion wise to a stirred solution of 0.83 g **9a** (2 mmol) in 10 cm³ dry *DMF* at room temp. To this resulting orange- colored solution, was added 0.85 g of iodomethane (6 mmol); the reaction mixture, which quickly acquired yellow coloration, was stirred for additional 15-20 min, and finally diluted with 70 cm³ cold water. The precipitated yellow solid was collected under suction, washed with 2 x 10 cm³ water, air-dried and recrystallized from CH₂Cl₂ / n-hexane. The IR and NMR spectral data of this product were found to be identical with those of 5a given above. Yield: 0.55 g (64 %); mp > 300°C (darkens at 240°C).

### Example 8

### Ethyl-1-cyclopropyl-6-fluoro-4,8-dioxo-4,8-dihydro-1H-indeno[2,1-b]pyrido[2,3-f] quinoxaline-3-carboxylate (1a)

A stirred mixture of indane-1,2,3-trione (0.29g, 1.6 mmol) and ethyl 7,8-diamino-1-cyclopropyl-6-fluoro-4-oxo-1,4-dihydroquinoline-3-carboxylate (0.50 g, 1.6 mmol) in absolute ethanol (40 ml) containing 0.2 mL of glacial acetic acid was brought to gentle reflux for 1 h. After cooling to rt, the resulting orange solid was filtered and washed with cold ethanol, the resulting two isomers were separated on silica gel TLC plates [MeOH / CHCl₃ (3:97, *v:v*)]. Yield of (**1a**) 0.27 g (38 %); mp 288-290 °C (decomp). Yield of (**2a**) 0.25 g (36 %);
mp 298-300 °C (decomp). IR (KBr) *v* : 3081, 2959, 2920, 2849, 1728, 1692, 1621, 1576, 1551, 1463, 1313, 1293, 1171, 1103, 1076, 1024 cm⁻¹. HRMS (ESI): calcd. for C₂₄H₁₇FN₃O₄⁺ [M+H]⁺: 430.12031, found: 430.11977; calcd. for C₂₄H₁₆FN₃O₄Na⁺ [M+Na]⁺ : 452.10225, found: 452.10167. ¹H NMR (300 MHz, CDCl₃): δ 1.01 (m, 2H) and 1.32 (m, 2H)(H₂-2' / H₂-3'), 1.43 (t, 3H, *J* = 7.1 Hz, C*H₃*CH₂O-), 4.43 (q, 2H, *J* = 7.1 Hz, -OC*H₂*CH₃), 4.80 (m, 1H, H-1'), 7.67 (dd, 1H, *J* = 7.1, 7.5 Hz, H-10), 7.81 (dd, 1H, *J* = 7.1, 7.6 Hz, H-11), 7.94 (d, 1H, *J* = 7.6 Hz, H-12), 7.98 (d, 1H, *J* = 7.5 Hz, H-9), 8.45(d, 1H, ³*J*_{H-F} = 9.7 Hz, H-5), 8.86 (s, 1H, H-2). ¹³C NMR (75 MHz, CDCl₃): δ 11.2 (C-2' / C-3'), 14.4 (CH₃), 42.7 (C-1'), 61.4 (-OCH₂Me), 111.6 (d, ²*J*_{C-F} = 20.9 Hz, C-5), 112.9 (C-3), 122.3 (C-12), 125.5 (C-9), 130.8 (d, ³J_{C-F} = 5.8 Hz, C-4a), 133.4 (C-10), 135.1 (C-13b), 136.1 (C-13a), 136.9 (C-8a), 137.0 (d, ²*J*_{C-F} = 10.6 Hz, C-6a), 137.1 (C-11), 140.8 (C-12a), 149.7 (d, ⁴*J*_{C-F} = 2 Hz, C-7a), 151.0 (C-2), 154.8 (C-12b), 155.7 (d, ¹*J*_{C-F} = 261 Hz, C-6), 165.2 (CO₂Et), 172.1 (d, ⁴*J*_{C-F} = 2 Hz, C-4), 188.2 (C-8).

### Example 9

### Ethyl-1-cyclopropyl-6-fluoro-4,12-dioxo-4,12-dihydro-1H-indeno[1,2-b]pyrido[2,3-f] quinoxaline-3-carboxylate (2a)

IR (KBr) *v*: 3068, 3010, 2920, 2849, 1736, 1698, 1621, 1576, 1454, 1381, 1293, 1255, 1203, 1184, 1158, 1101, 1017 cm⁻¹. HRMS (ESI): calcd. for C₂₄H₁₇FN₃O₄⁺ [M+H]⁺: 430.12031, found: 430.11970; calcd. for C₂₄H₁₆FN₃O₄Na⁺ [M+Na]⁺ : 452.10225, found: 452.10161. ¹H NMR (300 MHz, CDCl₃): δ 0.95 (m, 2H) and 1.34 (m, 2H)(H₂-2' / H₂-3'), 1.42 (t, 3H, *J* = 7.1 Hz, C*H₃*CH₂O-), 4.42 (q, 2H, *J* = 7.1 Hz, -OC*H₂*CH₃), 4.70 (m, 1H, H-1'), 7.67 (dd, 1H, *J* = 7.5, 7.2 Hz, H-10), 7.81 (dd, 1H, *J* = 7.4, 7.2 Hz, H-9), 7.93 (d, 1H, *J* = 7.5 Hz, H-11), 8.21 (d, 1H, *J* = 7.4 Hz, H-8), 8.51 (d, 1H, ³*J*_{H-F} = 10.1 Hz, H-5), 8.82 (s, 1H, H-2). ¹³C NMR (75 MHz, CDCl₃): δ 11.3 (C-2' / C-3'), 14.4 (CH₃), 42.8 (C-1'), 61.3 (-OCH₂Me), 113.0 (C-3), 113.9 (d, ²*J*_{C-F} = 21.3 Hz, C-5), 123.5 (C-8), 124.9 (C-11), 129.7 (d, ³*J*_{C-F} = 6.6 Hz, C-4a), 133.6 (C-10), 136.3 (d, ⁴*J*_{C-F} = 2.8 Hz, C-13b), 136.5 (C-13a), 136.6 (d, ²*J*_{C-F} = 14.5 Hz, C-6a), 137.1 (C-9), 137.2 (C-11a), 140.4 (C-7b), 150.8 (C-2), 151.0 (C-12a), 154.7 (d, ¹*J*_{C-F} = 258 Hz, C-6), 157.8 (d, ⁴*J*_{C-F} = 1.8 Hz, C-7a), 165.0 (*C*O₂Et), 172.1(d, ⁴*J*_{C-F} = 2 Hz, C-4), 188.7 (C-12).

### Example 10

### 1-Cyclopropyl-6-fluoro-4,8-dioxo-4,8-dihydro-1H-indeno[2,1-b]pyrido[2,3-f] quinoxaline-3-carboxylic acid (1b)

Ethyl 1-cyclopropyl-6-fluoro-4,8-dioxo-4,8-dihydro-1*H*-indeno[2,1-*b*]pyrido[2,3-*f*] quinoxaline-3-carboxylate (**1a**)(0.20 g, 0.47 mmol) was refluxed in 12% aqueous HCl (30 ml) and ethanol (10 ml), monitored by TLC and was completed within 20-24 h. Thereafter, the reaction mixture was cooled and the resulting precipitate was collected, washed with water and dried. Yield 0.18 g (94 %); mp > 300 °C. IR (KBr) *v*: 3447, 3068, 2959, 2926, 2850, 1731, 1634, 1615, 1584, 1551. 1465, 1313, 1106 cm⁻¹. HRMS (ESI): calcd. for C₂₂H₁₃FN₃O₄⁺ [M+H]⁺: 402.08901, found: 402.08846; calcd. for C₂₂H₁₂FN₃O₄Na⁺ [M+Na]⁺ : 424.07095, found: 424.07047. ¹H NMR (300 MHz, DMSO-d₆): δ 1.07 (m, 2H) and 1.19 (m, 2H) (H₂-2' / H₂-3'), 4.98 (m, 1H, H-1'), 7.76 (dd, 1H, *J* = 7.1, 7.5 Hz, H-10), 7.90 (dd, 1H, *J* = 7.1, 7.8 Hz, H-11), 7.95 (d, 1H, *J* = 7.8 Hz, H-12), 8.26 (d, 1H, *J* = 7.5 Hz, H-9), 8.31 (d, 1H, ³*J* _{H-F} = 9.9 Hz, H-5), 9.03 (s, 1H, H-2), 14.68 (s, 1H, CO*₂H*).

### Example 11

### 1-Cyclopropyl-6-fluoro-4,12-dioxo-4,12-dihydro-1H-indeno[1,2-b]pyrido [2,3-f] quinoxaline-3-carboxylic acid (2b)

Ethyl-1-cyclopropyl-6-fluoro-4,12-dioxo-4,12-dihydro-1*H*-indeno[1,2-*b*]pyrido[2,3-***f*]** quinoxaline-3-carboxylate (**2a**)(0.20 g, 0.47 mmol) was refluxed in 12% aqueous HCl (30 ml) and ethanol (10 ml), monitored by TLC and was completed within 20-24 h. Thereafter, the reaction mixture was cooled and the resulting precipitate was collected, washed with water and dried. Yield 0.18 g (94%); mp > 300 °C. IR (KBr) *v* : 3425, 3055, 2959, 2920, 2862, 1734, 1633, 1612, 1579, 1558, 1499, 1468, 1385, 1354, 1315, 1247, 1205, 1155, 1112, 1070 cm⁻¹. HRMS (ESI): calcd. for C₂₂H₁₃FN₃O₄⁺ [M+H]⁺: 402.08901, found: 402.08845; calcd. for C₂₂H₁₂FN₃O₄Na⁺ [M+Na]⁺ : 424.07095, found: 424.07045. ¹H NMR (300 MHz, DMSO-d₆): δ 0.95 (m, 2H) and 1.22 (m, 2H)(H₂-2' / H₂-3'), 4.70 (m, 1H, H-1'), 7.79 (dd, 1H, *J* = 7.1, 7.4 Hz, H-10), 7.93 (dd, 1H, *J* = 7.4, 7.5 Hz, H-9), 7.96 (d, 1H, *J* = 7.1 Hz, H-11), 8.20 (d, 1H, *J* = 7.5 Hz, H-8), 8.39(d, 1H, ³*J*_{H-F} = 10.0 Hz, H-5), 8.98 (s, 1H, H-2), 14.68 (s, 1H, CO₂*H*).

### Elemental Analyses (in Tabulated form) for refereeing purposes

**Compound 5:** Anal. Calcd for C₂₄H₁₉FN₄O₃: C, 66.97; H, 4.45; N,13.02. Found: C, 66.82; H, 4.41; N, 12.87.
**Compound 6**: Anal. Calcd for C₂₄H₁₉FN₄O₃: C, 66.97; H, 4.45; N,13.02. Found: C, 66.87; H, 4.43; N, 12.81.
**Compound 9:** Anal. Calcd for C₂₃H₁₇FN₄O₃: C, 66.34; H, 4.11; N,13.45. Found: C, 66.18; H, 4.03; N, 13.32.
**Compound 10**: Anal. Calcd for C₂₃H₁₆ClFN₄O₃: C, 61.27; H, 3.58; Cl, 7.86; N,12.43. Found: C, 61.02; H, 3.44; Cl, 7.63; N, 12.25.

- Figure 1:: An ORTEP plot of the molecular structure of 5 showing the arbitrary crystallographic numbering scheme, being different from the IUPAC numbering adopted in Scheme 2. Displacement ellipsoids are drawn at the 50 % probability level, while H atoms are shown as spheres of arbitrary radii.
- Figure 2: Packing diagram for 5 in the unit cell (z = 8) / viewed along the c- axis.
- Figure 3: Centrosymmetric pair wise arranged molecules for 5 in the crystal, linked via C-H···O bonds.

## Claims

1. Quinoxaline derivative **characterised in that** the quinoxaline is represented by general formulas I or II, 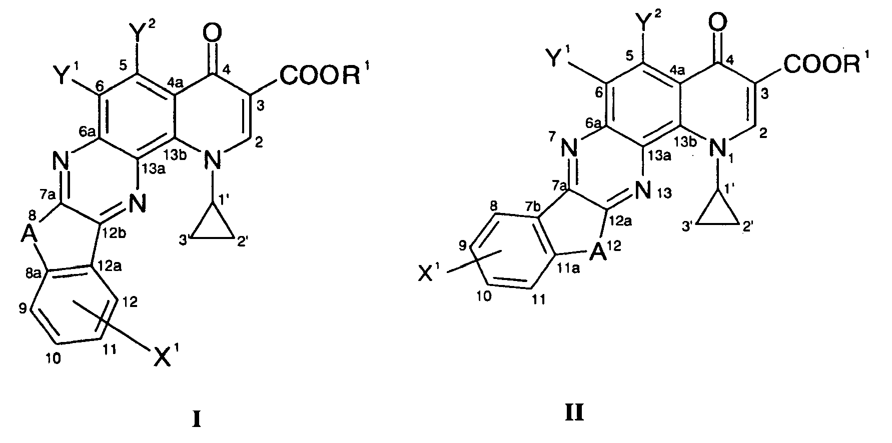
- wherein COOR¹ independently is an acid, a salt and/or an ester, wherein in the ester R¹ is independently a linear or branched C₁-C₆-alkyl,
- Y¹ and/or Y² are independently halogen and/or H, in particular F, Cl or H,
- A is oxo (C=O), C(R³)₂ and/or N-R², wherein each R³ and/or R² independently are H, a linear, branched or cyclic C₁-C₆-alkyl or C₁-C₁₂-alkylamin, unsubstituted or substituted hydrocarbon and
- X¹ is H, a linear, branched or cyclic unsubstituted or substituted hydrocarbon or halogen, in particular F, Cl, Br or I and/or a polymorph or a solvate of the quinoxaline.

2. Quinoxaline according to claim 1, wherein in general formulas I and/or II R¹ independently is H, a linear or branched C₁-C₆-alkyl, in particular methyl, ethyl, iso-propyl, n-propyl, butyl, n-butyl, iso-butyl, tert-butyl, an alkali and/or alkaline earth metal, Y¹ is F, Cl, Br or I; A is oxo (C=O), C(R³)₂ and/or N-R², wherein each R³ and/or R² independently are H, linear, branched or cyclic C₁-C₆-alkyl or C₁-C₁₂-alkylamin and X¹ is H, F, Cl, Br or I.

3. Quinoxaline according to claim 1 or 2, wherein in formulas I and/or II R¹ is H, methyl, ethyl or an alkali metal, Y¹ is F, Y² is H and A is C=O or N-R², wherein R² is H, methyl or CH₂CH₂N(CH₃)₂ and X¹ is H or Cl.

4. Quinoxaline according to one of claims 1 to 3, wherein in formulas I and/or II R¹ is H, methyl, ethyl or alkali metal, Y¹ is F, Y² is H and A is N-R², wherein R² is H, methyl or CH₂CH₂N(CH₃)₂ and X¹ is H or Cl and X¹ is attached to C-11 in formula I or X¹ is attached to C-9 in formula II.

5. Quinoxaline according to one of claims 1 to 3, wherein in formulas I and/or II R¹ is H, methyl, ethyl or alkali metal, Y¹ is F, Y² is H and A is C=O and X¹ is H.

6. Quinoxaline according to one of claims 1 to 5, wherein the quinoxaline of formulas I and/or II is selected from ethyl 11-chloro-1-cyclopropyl-6-fluoro-4-oxo-4,8-dihydro-1H-indolo[3,2-b]pyrido [2,3-f]quinoxaline-3-carboxylate, ethyl-1-cyclopropyl-6-fluoro-4-oxo-4,8-dihydro-1H-indolo[3,2-b]pyrido[2,3-f]quinoxaline-3-carboxylate, ethyl 1-cyclopropyl-6-fluoro-12-methyl-4-oxo-4,12-dihydro-1H-indolo[2,3-b]pyrido [2,3-f]quinoxaline-3-carboxylate, ethyl-1-cyclopropyl-6-fluoro-8-methyl-4-oxo-4,8-dihydro-1H-indolo[3,2-b]pyrido [2,3-f] quinoxaline-3-carboxylate, ethyl-7,8-diamino-1-cyclopropyl-6-fluoro-4-oxo-1,4-dihydroquinoline-3-carboxylate, 1-cyclopropyl-6-fluoro-8-methyl-4-oxo-4,8-dihydro-1H-indolo [2,3-b]pyrido[2,3-f]quinoxaline-3-carboxylic acid, 1-cyclo propyl-6-fluoro-4-oxo-4,8-dihydro-1H-indolo[2,3-b]pyrido[2,3-f]quinoxaline-3-carboxy acid, 11-chloro-1-cyclopropyl-6-fluoro-4-oxo-4,8-dihydro-1H-indolo[2,3-b]pyrido [2,3-f]quinoxaline-3-carboxy acid, ethyl 1-cyclopropyl-6-fluoro-4,8-dioxo-4,8-dihydro-1H-indeno[2,1-b]pyrido[2,3-f]quinoxaline-3-carboxylate, ethyl 1-cyclopropyl-6-fluoro-4,12-dioxo-4,12-dihydro-1H-indeno[1,2-b]pyrido[2,3-f] quinoxaline-3-carboxylate, 1-cyclopropyl-6-fluoro-4,8-dioxo-4,8-dihydro-1H-indeno[2,1-b]pyrido[2,3-f] quinoxaline-3-carboxylic acid and/or 1-cyclopropyl-6-fluoro-4,12-dioxo-4,12-dihydro-1H-indeno[1,2-b]pyrido[2,3-*f*] quinoxaline-3-carboxylic acid.

7. Synthesis for preparing quinoxalines represented by general formulas I and/or II, 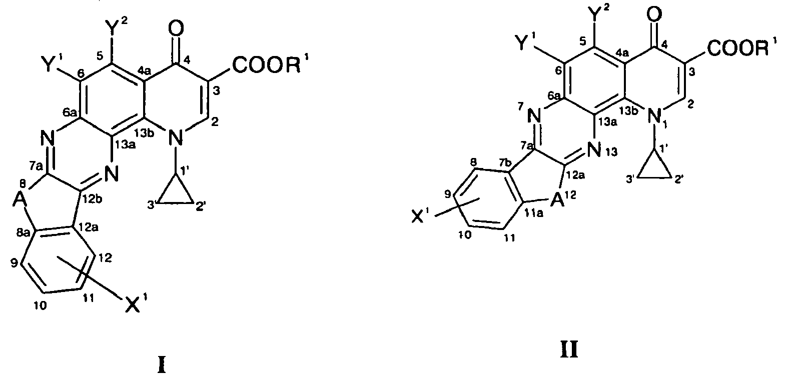
- wherein COOR¹ independently is an acid, an ester and/or a salt,
- Y¹ and/or Y² are independently halogen and/or H, in particular F, Cl or H,
- A is oxo (C=O), C(R³)₂ and/or N-R², wherein each R³ and/or R² independently are H, linear, branched or cyclic C₁-C₆-alkyl or C₁-C₁₂-alkylamin and
- X¹ is H, a linear, branched or cyclic unsubstituted or substituted hydrocarbon or halogen, in particular F, Cl, Br or I;
- wherein a key precursor of general formula IV and wherein Y¹, Y² and R¹ are defined as above is reacted 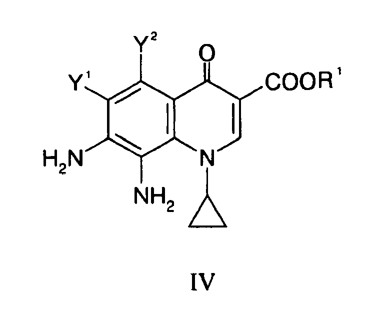
- with a substance derivative of general formula III 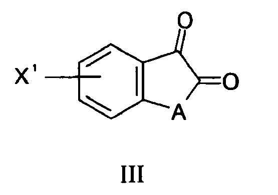
- wherein A and X¹ are as defined above, optional in presence of an acid.

8. Synthesis according to claim 7, wherein R¹ independently is H, a linear or branched C₁-C₆-alkyl, in particular methyl, ethyl, iso-propyl, n-propyl, butyl, n-butyl, iso-butyl, tert-butyl, an alkali and/or alkaline earth metal.

9. Synthesis according to one of claims 6 to 8, wherein the acid is polyphosphoric acid.

10. Key precursor of general formula IV 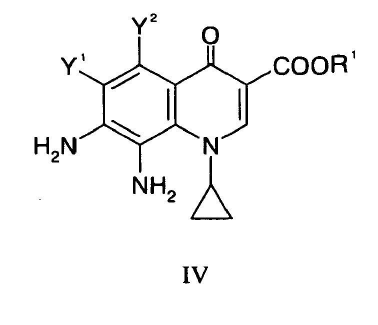
- wherein COOR¹ forms independently an acid, an ester and/or a salt,
- Y¹ and/or Y² are independently halogen and/or H, in particular F, Cl or H, and/or a polymorph or a solvate of the precursor, in particular for the synthesis of quinoxaline of general formulas I and/or II.

11. Key precursor according to claim 10, wherein R¹ is H, Y¹ is fluorine and Y² is H; R¹ is methyl, Y¹ is fluorine and Y² is H;, R¹ is ethyl, Y¹ is fluorine and Y² is H; R¹ is Na⁺ or Ca²⁺ ,Y¹ is fluorine and Y² is H; R¹ is propyl,Y¹ is fluorine and Y² is H; or R¹ is butyl, Y¹ is fluorine and Y² is H.

12. Synthesis for preparing the key precursor according to claim 10 or 11 by reduction of a substance of general formula V 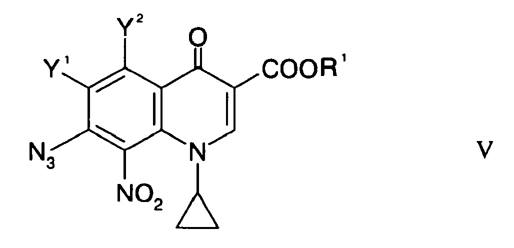
- wherein COOR¹ forms independently an acid, an ester and/or a salt, Y¹ and/or Y² are independently halogen and/or H, in particular F, Cl or H.

13. Quinoxaline of general formulas I and/or II according to one of claims 1 to 9 for use as pharmaceutical active ingredient.

14. Quinoxaline of general formulas I and/or II according to one of previous claims 1 to 9 for use as an agent having effect against cancer, as an antibiotic and/or as antiviral agent.

15. Pharmaceutical, food, animal feed or cosmetic formulation comprising quinoxaline derivatives of general formula I and/or II of one of the previous claims 1 to 9 and at least one pharmaceutical, food, animal feed and/or cosmetic excipient.

## Patentansprüche

1. Quinoxalin Derivate **dadurch gekennzeichnet, dass** die Quinoxalin Derivate der allgemeinen Formel I oder II entsprechen, 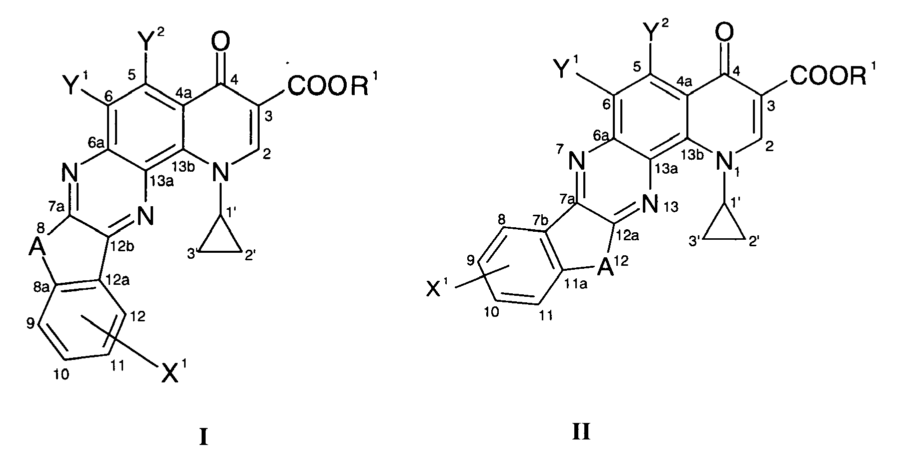
- wobei COOR¹ unabhängig voneinander eine Säure, ein Salz und/oder ein Ester ist, und R¹ im Ester unabhängig voneinander eine lineare oder verzweigte C₁-C₆-Alkyl-Gruppe ist,
- Y¹ und/oder Y² unabhängig voneinander Halogen und/oder H sind, insbesondere F, Cl oder H,
- A ist oxo (C=O), C(R³)₂ und/oder N-R², wobei jedes R³ und/oder R² unabhängig voneinander H, ein lineares, verzweigtes oder cyclisches C₁-C₆-Alkyl- oder C₁-C₁₂-Alkylamin, unsubstituierter oder substituierter Kohlenwasserstoff ist und
- X¹ ist H, ein linearer, verzweigter oder cyclischer unsubstituierter oder substituierter Kohlenwasserstoff oder Halogen, insbesondere F, Cl, Br oder J und/oder ein Polymorph oder Solvat des Quinoxalins.

2. Quinoxalin nach Anspruch 1, **dadurch gekennzeichnet, dass** in der allgemeinen Formel I und/oder II R¹ unabhängig voneinander H, eine lineare oder verzweigte C₁-C₆-Alkyl-Gruppe, insbesondere Methyl, Ethyl, iso-Propyl, n-Propyl, Butyl, n-Butyl, iso-Butyl, tert-Butyl, ein Alkali- und/oder Erdalkalimetall ist, Y¹ ist F, Cl, Br oder J; A ist oxo (C=O), C(R³)₂ und/oder N-R², wobei jedes R³ und/oder R² unabhängig voneinander H, eine lineare, verzweigte oder cyclische C₁-C₆-Alkyl- oder C₁-C₁₂-Alkylamin-Gruppe ist und X¹ ist H, F, Cl, Br oder J.

3. Quinoxalin nach Anspruch 1 oder 2, **dadurch gekennzeichnet, dass** in den allgemeinen Formeln I und/oder II R¹ einem H, Methyl, Ethyl oder einem Alkalimetall entspricht, Y¹ ist F, Y² ist H und A ist C=O oder N-R², wobei R² ein H, Methyl oder CH₂CH₂N(CH₃)₂ entspricht und X¹ ist H oder Cl.

4. Quinoxalin nach einem der Ansprüche 1 bis 3, **dadurch gekennzeichnet, dass** in den allgemeinen Formeln I und/oder II R¹ einem H, Methyl, Ethyl oder Alkalimetall entspricht, Y¹ ist F, Y² ist H und A ist N-R², wobei R² einem H, Methyl oder CH₂CH₂N(CH₃)₂ entspricht und X¹ ist H oder Cl und X¹ in Formel I an C-11 gebunden ist oder X¹ in Formel II an C-9 gebunden ist.

5. Quinoxalin nach einem der Ansprüche 1 bis 4, **dadurch gekennzeichnet, dass** in den allgemeinen Formeln I und/oder 11 R¹ einem H, Methyl, Ethyl oder Alkalimetall entspricht, Y¹ ist F, Y² ist H und A ist C=O und X¹ ist H.

6. Quinoxalin nach einem der Ansprüche 1 bis 5, **dadurch gekennzeichnet, dass** das Quinoxalin in den allgemeinen Formeln I und/oder II ausgewählt ist aus Ethyl-11-chlor-1-cyclopropyl-6-fluor-4-oxo-4,8-dihydro-1H-indolo[3,2-b]pyrido [2,3-f]quinoxalin-3-carboxylat, Ethyl-1-cyclopropyl-6-fluor-4-oxo-4,8-dihydro-1H-indolo[3,2-b]pyrido[2,3-f]quinoxalin-3-carboxylat, Ethyl-1-cyclopropyl-6-fluor-12-methyl-4-oxo-4,12-dihydro-1H-indolo[2,3-b]pyrido [2,3-f]quinoxalin-3-carboxylat, Ethyl-1-cyclopropyl-6-fluor-8-methyl-4-oxo-4,8-dihydro-1H-indolo[3,2-b]pyrido [2,3-f] quinoxalin-3-carboxylat, Ethyl-7,8-diamino-1-cyclopropyl-6-fluor-4-oxo-1,4-dihydroquinolin-3-carboxylat, 1-Cyclopropyl-6-fluor-8-methyl-4-oxo-4,8-dihydro-1H-indolo[2,3-b]pyrido[2,3-f]quinoxalin-3-carbonsäure, 1-Cyclopropyl-6-fluor-4-oxo-4,8-dihydro-1H-indolo[2,3-b]pyrido [2,3-f]quinoxalin-3-carbonsäure, 11-Chlor-1-cyclopropyl-6-fluor-4-oxo-4,8-dihydro-1H-indolo[2,3-b]pyrido[2,3-f]quinoxalin-3-carbonsäure, Ethyl 1-cyclopropyl-6-fluor-4,8-dioxo-4,8-dihydro-1H-indeno[2,1-b]pyrido[2,3-f] quinoxalin-3-carboxylat, Ethyl-1-cyclopropyl-6-fluor-4,12-dioxo-4,12-dihydro-1H-indeno[1,2-b]pyrido[2,3-f]quinoxalin-3-carboxylat, 1-cyclopropyl-6-fluoro-4,8-dioxo-4,8-dihydro-1H-indeno[2,1-b]pyrido[2,3-f]quinoxalin-3-carbonsäue und/oder 1-Cyclopropyl-6-fluor-4,12-dioxo-4,12-dihydro-1H-indeno[1,2-b]pyrido [2,3-*f*]quinoxalin-3-carbonsäure.

7. Verfahren zur Herstellung von Quinoxalinen der allgemeinen Formeln I und/oder II, 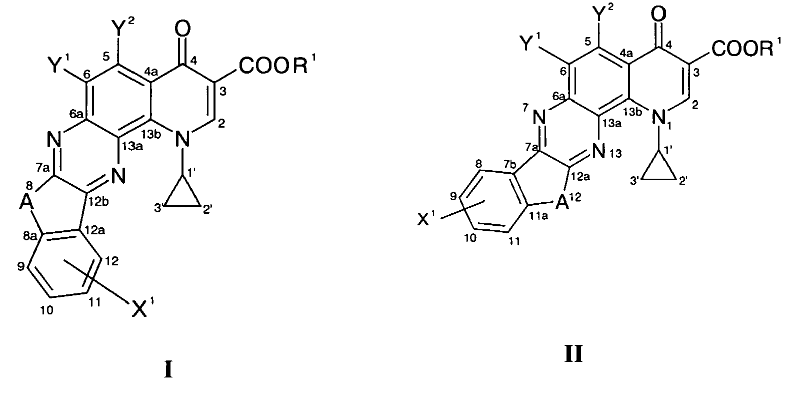
- wobei COOR¹ unabhängig voneinander eine Säure, ein Salz und/oder ein Ester ist,
- Y¹ und/oder Y² unabhängig voneinander Halogen und/oder H sind, insbesondere F, Cl oder H,
- A ist oxo (C=O), C(R³)₂ und/oder N-R², wobei jedes R³ und/oder R² unabhängig voneinander H, eine lineare, verzweigte oder cyclische C₁-C₆-Alkyl- oder C₁-C₁₂-Alkylamin ist und
- X¹ ist H, ein linearer, verzweigter oder cyclischer unsubstituierter oder substituierter Kohlenwasserstoff oder Halogen, insbesondere F, Cl, Br oder J;
- in dem ein Zwischenprodukt der allgemeinen Formel IV, mit Y¹, Y² und R¹ wie vorstehend definiert mit einer 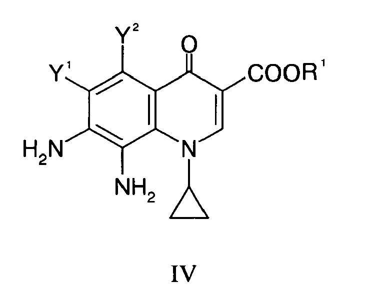
- Verbindung der allgemeinen Formel III umgesetzt wird 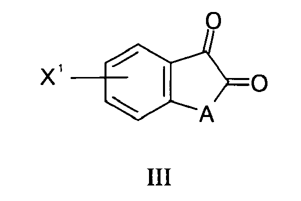
- worin A und X¹ wie vorstehend definiert sind, wobei die Umsetzung optional in Gegenwart einer Säure erfolgt.

8. Verfahren nach Anspruch 7, **dadurch gekennzeichnet, dass** R¹ unabhängig H, eine lineare oder verzweigte C₁-C₆-Alkyl-Gruppe, insbesondere Methyl, Ethyl, iso-Propyl, n-Propyl, Butyl, n-Butyl, iso-Butyl, tert-Butyl, ein Alkalimetall und/oder ein Erdalkalimetall ist.

9. Verfahren nach einem der Ansprüche 6 bis 8, **dadurch gekennzeichnet, dass** die Säure Polyphosphorsäure ist.

10. Zwischenprodukt der allgemeinen Formel IV 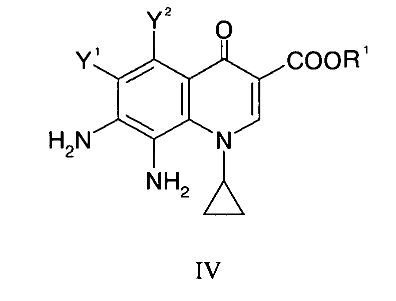
- worin COOR¹ unabhängig eine Säure, ein Ester und/oder ein Salz ist,
- Y¹ und/oder Y² unabhängig voneinander Halogen und/oder H sind, insbesondere F, Cl oder H, und/oder ein Polymorph oder ein Solvate des Zwischenproduktes, insbesondere zur Herstellung eines Quinoxalins der allgemeinen Formel I und/oder II.

11. Zwischenprodukt nach Anspruch 10, worin R¹ ein H ist, Y¹ ist Fluor und Y² ist H; R¹ ist Methyl, Y¹ ist Fluor und Y² ist H; R¹ ist Ethyl, Y¹ ist Fluor und Y² ist H; R¹ ist Na⁺ oder Ca²⁺, Y¹ ist Fluor und Y² ist H; R¹ ist Propyl,Y¹ ist Fluor und Y² ist H; oder R¹ ist Butyl, Y¹ ist Fluor und Y² ist H.

12. Verfahren zur Herstellung des Zwischenproduktes nach Anspruch 10 oder 11 mittels Reduzierung einer Verbindung der allgemeinen Formel V 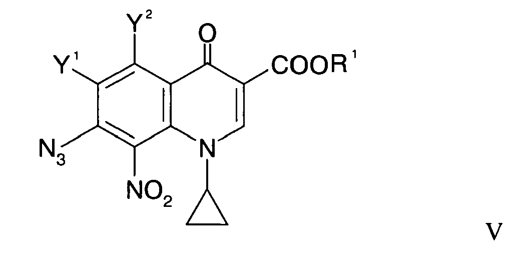
- worin COOR¹ unabhängig eine Säure, ein Ester und oder ein Salz ist,
Y¹ und/oder Y² unabhängig Halogen und/oder H sind, insbesondere F, Cl oder H.

13. Quinoxalin der allgemeinen Formeln I und/oder II nach einem der Ansprüche 1 bis 9 zur Verwendung als Arzneimittel.

14. Quinoxalin der allgemeinen Formeln I und/oder II nach einem der vorstehenden Ansprüche 1 bis 9 zur Verwendung als Mittel zur Behandlung von Krebs, als Antibiotikum und/oder als Virostatikum.

15. Pharmazeutische, Lebensmittel- oder Futtermittelformulierung umfassend Quinoxalin Derivate der allgemeinen Formel I und/oder II nach einem der vorstehenden Ansprüche 1 bis 9 und mindestens ein pharmazeutisches Hilfsmittel, einen Lebensmittelhilfsstoff, einen Futtermittelhilfsstoff und/oder ein kosmetisches Hilfsmittel.

## Revendications

1. Dérivé de quinoxaline, **caractérisé en ce que** la quinoxaline répond à la formule générale I ou II, 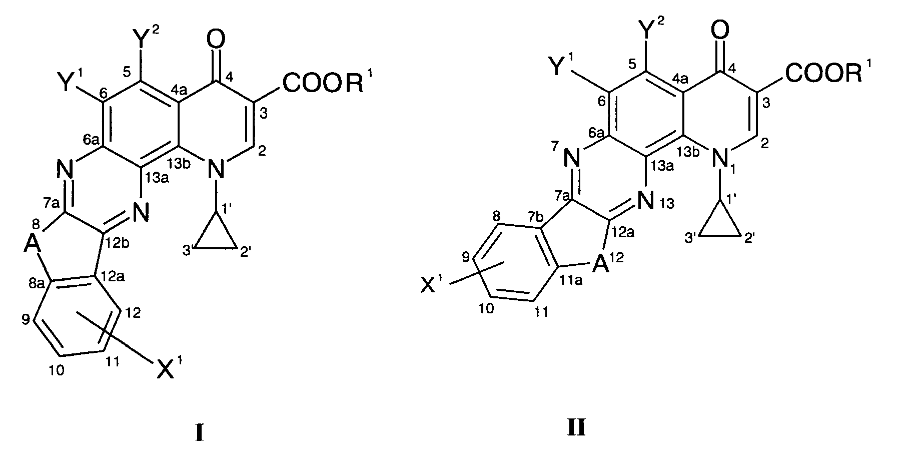
- dans laquelle COOR¹ représente indépendamment un acide, un sel et/ou un ester, dans lequel R¹ représente indépendamment un C₁-C₆-alkyle linéaire ou ramifié,
- Y¹ et/ou Y² représentent indépendamment un atome d'halogène et/ou un atome d'hydrogène, en particulier F, Cl ou H,
- A représente un groupe oxo (C=O), C(R³)₂ et/ou N-R², dans lesquels chaque R³ et/ou R² représentent indépendamment un atome d'hydrogène, un C₁-C₆-alkyle linéaire, ramifié ou cyclique ou un C₁-C₁₂-alkylamine, un hydrocarbure non substitué ou substituée et
- X¹ représente un atome d'hydrogène, un hydrocarbure linéaire, ramifié ou cyclique non substitué ou substitué ou un atome d'halogène, en particulier F, Cl, Br ou I et/ou un polymorphe ou un solvate de la quinoxaline.

2. Quinoxaline suivant la revendication 1 répondant aux formules générales I et/ou II, dans laquelle R¹ représente indépendamment un atome d'hydrogène, un C₁-C₆-alkyle linéaire ou ramifié, en particulier un groupe méthyle, éthyle, isopropyle, n-propyle, butyle, n-butyle, isobutyle, tert-butyle, un métal alcalin et/ou alcalino-terreux, Y¹ représente un atome de fluor, un atome de chlore, un atome de brome ou un atome de iode; A représente un groupe oxo (C=O), C(R³)₂ et/ou N-R², dans lesquels chaque R³ et/ou R² représentent indépendamment un atome d'hydrogène, un C₁-C₆-alkyle linéaire, ramifié ou cyclique ou un C₁-C₁₂-alkylamine et X¹ représente un atome d'hydrogène, un atome de fluor, un atome de chlore, un atome de brome ou un atome de iode.

3. Quinoxaline suivant les revendications 1 ou 2 répondant aux formules générales I et/ou II, dans laquelle R¹ représente un atome d'hydrogène, un groupe méthyle, éthyle ou un métal alcalin, Y¹ représente un atome de fluor, Y² représente un atome d'hydrogène et A représente un groupe C=O ou N-R², dans lequel R² représente un atome d'hydrogène, un groupe méthyle ou CH₂CH₂N(CH₃)₂ et X¹ représente un atome d'hydrogène ou un atome de chlore.

4. Quinoxaline suivant une des revendications 1 à 3 répondant aux formules I et/ou II, dans laquelle R¹ représente un atome d'hydrogène, un groupe méthyle, éthyle ou un métal alcalin, Y¹ représente un atome de flour, Y² représente un atome d'hydrogène et A représente un groupe N-R², dans lequel R² représente un atome d'hydrogène, un groupe méthyle ou CH₂CH₂N(CH₃)₂ et X¹ représente un atome d'hydrogène ou un atome de chlore et X¹ est rattaché à C-11 dans la formule I ou X¹ est rattaché à C-9 dans la formule II.

5. Quinoxaline suivant une des revendications 1 à 3 répondant aux formules I et/ou II, dans laquelle R¹ représente un atome d'hydrogène, un groupe méthyle, éthyle ou un métal alcalin, Y¹ représente un atome de fluor, Y² représente un atome d'hydrogène et A représente C=O et X¹ représente un atome d'hydrogène.

6. Quinoxaline suivant une des revendications 1 à 5, dans laquelle la quinoxaline répondant aux formules I et/ou II est choisi parmi éthyl 11-chloro-1-cyclopropyl-6-fluoro-4-oxo-4,8-dihydro-1H-indolo[3,2-b]pyrido [2,3-f]quinoxaline-3-carboxylate, éthyl-1-cyclopropyl-6-fluoro-4-oxo-4,8-dihydro-1H-indolo[3,2-b]pyrido[2,3-f]quinoxaline-3-carboxylate, éthyl 1-cyclopropyl-6-fluoro-12-méthyl-4-oxo-4,12-dihydro-1H-indolo[2,3-b]pyrido[2,3-f]quinoxaline-3-carboxylate, éthyl-1-cyclopropyl-6-fluoro-8-méthyl-4-oxo-4,8-dihydro-1H-indolo[3,2-b]pyrido [2,3-f] quinoxaline-3-carboxylate, éthyl-7,8-diamino-1-cyclopropyl-6-fluoro-4-oxo-1,4-dihydroquinoline-3-carboxylate, acide 1-cyclopropyl-6-fluoro-8-méthyl-4-oxo-4,8-dihydro-1H-indolo [2,3-b]pyrido[2,3-f]quinoxaline-3-carboxylique, acide 1-cyclo propyl-6-fluoro-4-oxo-4,8-dihydro-1H-indolo[2,3-b]pyrido[2,3-f]quinoxaline-3-carboxylique, acide 11-chloro-1-cyclopropyl-6-fluoro-4-oxo-4,8-dihydro-1H-indolo[2,3-b]pyrido [2,3-f]quinoxaline-3-carboxylique, éthyl 1-cyclopropyl-6-fluoro-4,8-dioxo-4,8-dihydro-1H-indeno[2,1-b]pyrido[2,3-f]quinoxaline-3-carboxylate, éthyl 1-cyclopropyl-6-fluoro-4,12-dioxo-4,12-dihydro-1H-indeno[1,2-b]pyrido[2,3-f] quinoxaline-3-carboxylate, acide 1-cyclopropyl-6-fluoro-4,8-dioxo-4,8-dihydro-1H-indeno[2,1-b]pyrido[2,3-f] quinoxaline-3-carboxylique et/ou acide 1-cyclopropyl-6-fluoro-4,12-dioxo-4,12-dihydro-1H-indeno[1,2-b]pyrido[2,3-*f*] quinoxaline-3-carboxylique.

7. Procédé pour la préparation des quinoxalines répondant aux formules générales I et/ou II, 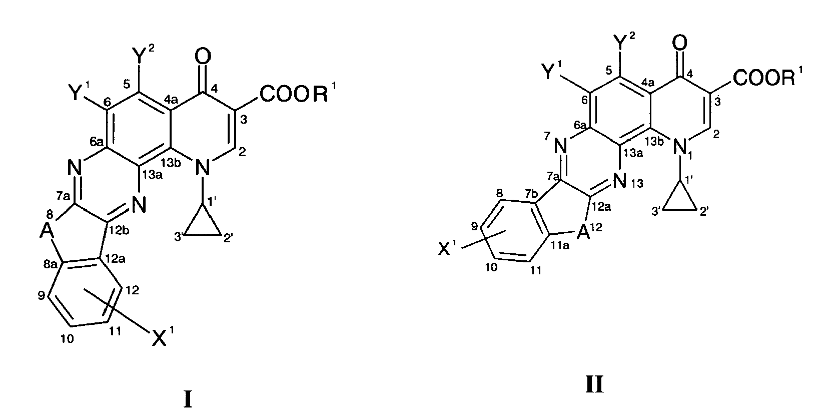
- dans lesquelles COOR¹ représente indépendamment un acide, un ester et/ou un sel,
- Y¹ et/ou Y² représentent indépendamment un atome d'halogène et/ou un atome d'hydrogène, en particulier F, Cl ou H,
- A représente un groupe oxo (C=O), C(R³)₂ et/ou N-R², dans lesquels chaque R³ et/ou R² représente indépendamment un atome d'hydrogène, un C₁-C₆-alkyle linéaire, ramifié ou cyclique ou un C₁-C₁₂-alkylamine et
- X¹ représente un atome d'hydrogène, un hydrocarbure linéaire, ramifié ou cyclique non substitué ou substitute ou un atome d'halogène, en particulier F, Cl, Br ou I;
- dans lequel on fait réagir un intermédiaire clé de la formule générale IV et dans laquelle Y¹, Y² et R¹ sont défini comme ci-dessus 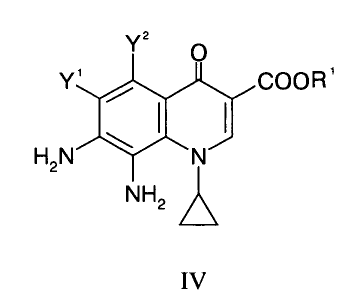
- avec un dérivé de la substance répondant à la formule générale III 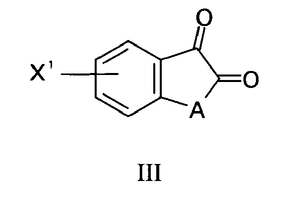
- dans laquelle A et X¹ sont défini comme ci-dessus, optionnellement en présence d'un acide.

8. Procédé suivant la revendication 7, dans lequel R¹ représente indépendamment un atome d'hydrogène, un C₁-C₆-alkyle linéaire ou ramifié, en particulier un groupe méthyle, éthyle, iso-propyle, n-propyle, butyle, n-butyle, iso-butyle, tert-butyle, un métal alcalin et/ou alcalino-terreux.

9. Procédé suivant une des revendications 6 à 8, dans lequel l'acide est un acide polyphosphorique.

10. Intermédiaire clé répondant à la formule générale IV 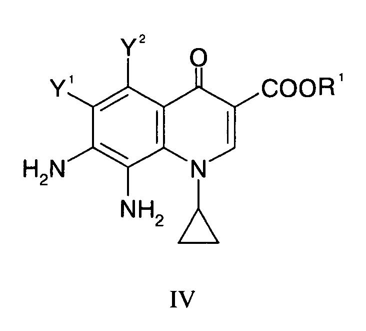
- dans laquelle COOR¹ forme indépendamment un acide, un ester et/ou un sel,
- Y¹ et/ou Y² représentent indépendamment un atome d'halogène et/ou un atome d'hydrogène, en particulier F, Cl ou H, et/ou un polymorphe ou un solvate de l'intermédiaire clé, en particulier pour la synthèse de la quinoxaline répondant aux formules générales I et/ou II.

11. Intermédiaire clé suivant la revendication 10, dans lequel R¹ représente un atome d'hydrogène, Y¹ représente un atome de fluor et Y² représente un atome d'hydrogène; R¹ représente un groupe méthyle, Y¹ représente un atome de fluor et Y² représente un atome d'hydrogène; R¹ représente un groupe éthyle, Y¹ représente un atome de fluor et Y² représente un atome d'hydrogène; R¹ représente Na⁺ ou Ca²⁺ ,Y¹ représente un atome de fluor et Y² représente un atome d'hydrogène; R¹ représente un groupe propyle,Y¹ représente un atome de fluor et Y² représente un atome d'hydrogène; ou R¹ représente un groupe butyle, Y¹ représente un atome de fluor et Y² représente un atome d'hydrogène.

12. Procédé pour la préparation de l'intermédiaire clé suivant les revendications 10 ou 11 par la réduction de la substance répondant à la formule générale V 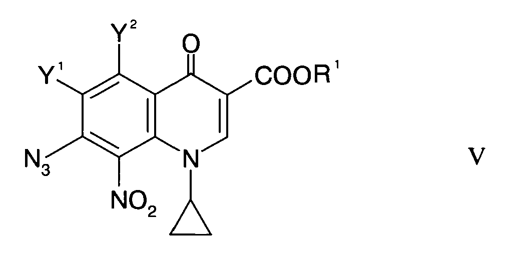
- dans laquelle COOR¹ forme indépendamment un acide, un ester et/ou un sel, Y¹ et/ou Y² représentent indépendamment un atome d'halogène et/ou un atome d'hydrogène, en particulier F, Cl ou H.

13. Quinoxaline répondant aux formules générales I et/ou II suivant une des revendications 1 à 9 pour l'utilisation comme ingrédient pharmaceutique actif.

14. Quinoxaline répondant aux formules générales I et/ou II suivant une des revendications précédentes 1 à 9 pour l'utilisation comme remède ayant un effet contre cancer, comme antibiotique et/ou comme remède antiviral.

15. Formulation pharmaceutique, alimentaire, des aliments pour animaux ou cosmétique comprenant des dérivés de quinoxaline répondant aux formules générales I et/ou II d'une des revendications précédentes 1 à 9 et au moins un excipient pharmaceutique, alimentaire, des aliments pour animaux et/ou cosmétique.
